# EUROPEAN PATENT APPLICATION

(11) **EP 2 031 066 A1**
(43) Date of publication of application: **04.03.2009**
(21) Application number: 07767052.9
(22) Date of filing: 05.06.2007
(51) Int. Cl.: C12N 15/54, C12N 1/15, C12N 1/19, C12N 1/21, C12N 5/10, C12N 9/10, C12P 21/02

(54) **MUTANT AND GENE ENCODING THE SAME**

(30) Priority: 08.06.2006 JP 2006160175
(71) Applicant: Nihon Shokuhin Kako Co., Ltd., Shibuya-ku, Tokyo 151-0051 (JP); Japan Agency for Marine-Earth Science and Technology, Yokosuka-shi Kanagawa 237-0061 (JP)
(72) Inventor: NAKAGAWA, Yoshinori, Shizuoka 4170031 (JP); TAKADA, Masayasu, Shizuoka 4170031 (JP); OGAWA, Koichi, Shizuoka 4170031 (JP); HATADA, Yuji, Kanagawa 2370061 (JP); HORIKOSHI, Hiroki, 2-15, Kanagawa 2370061 (JP)
(74) Representative: Meyers, Hans-Wilhelm
(86) International application number: PCT/JP2007/061391
(87) International publication number: WO 2007/142243

(57) **Abstract**

A mutant CGTase, having greater specific activity in the synthesis of *gamma-*cyclodextrin than the parent *gamma*-CGTase, in which at least one of the amino acid residues in the parent *gamma*-CGTase has been replaced with an amino acid residue differing from that particular amino acid residue in the parent *gamma*-CGTase. A gene encoding the amino acid sequence of the mutant. A method for manufacturing the mutant CGTase comprising: culturing the transformant that has been transformed or modified by chromosomal homologous recombination with the recombinant vector comprising the gene, and collecting the mutant CGTase produced by the transformant. Provided is an improved mutant CGTase that is imparted with greater specific activity than the parent *gamma*-CGTase by the addition of a mutation to *gamma*-CGTase, reducing the quantity of enzyme required to obtain a targeted CD composition. Provided is a gene encoding this mutant CGTase and a method for manufacturing the mutant CGTase employing this gene.

SEQUENCE LISTING
<110> Nihon Shokuhin Kako Co., Ltd. ; Japan Agency for Marine-Earth Science and
Technology
<120>ƒÁ-Cyclodextrin glucanotransferase mutants and genes coding the same
<130> A75107H
<160> 50

<210> 1
<211> 674
<212> PRT
<213> Bacillus clarkii 7364
<400> 1
Ser Asn Ala Thr Asn Asp Leu Ser Asn Val Asn Tyr Ala Glu Glu Val
1               5                   10                  15
Ile Tyr His Ile Val Thr Asp Arg Phe Lys Asp Gly Asp Pro Asp Asn
            20                  25                  30
Asn Pro Gln Gly Gln Leu Phe Ser Asn Gly Cys Ser Asp Leu Thr Lys
        35                  40                  45
Tyr Cys Gly Gly Asp Trp Gln Gly Ile Ile Asp Glu Ile Glu Ser Gly
    50                  55                  60
Tyr Leu Pro Asp Met Gly Ile Thr Ala Leu Trp Ile Ser Pro Pro Val
65                  70                  75                  80
Glu Asn Val Phe Asp Leu His Pro Glu Gly Phe Ser Ser Tyr His Gly
                85                  90                  95
Tyr Trp Ala Arg Asp Phe Lys Lys Thr Asn Pro Phe Phe Gly Asp Phe
            100                 105                 110
Asp Asp Phe Ser Arg Leu Ile Glu Thr Ala His Ala His Asp Ile Lys
        115                 120                 125
Val Val Ile Asp Phe Val Pro Asn His Thr Ser Pro Val Asp Ile Glu
    130                 135                 140
Asp Gly Ala Leu Tyr Asp Asn Gly Thr Leu Leu Gly His Tyr Ser Thr
145                 150                 155                 160
Asp Ala Asn Asn Tyr Phe Tyr Asn Tyr Gly Gly Ser Asp Phe Ser Asp
                165                 170                 175
Tyr Glu Asn Ser Ile Tyr Arg Asn Leu Tyr Asp Leu Ala Ser Leu Asn
            180                 185                 190
Gln Gln His Ser Phe Ile Asp Lys Tyr Leu Lys Glu Ser Ile Gln Leu
        195                 200                 205
Trp Leu Asp Thr Gly Ile Asp Gly Ile Arg Val Asp Ala Val Ala His
    210                 215                 220
Met Pro Leu Gly Trp Gln Lys Ala Phe Ile Ser Ser Val Tyr Asp Tyr
225                 230                 235                 240
Asn Pro Val Phe Thr Phe Gly Glu Trp Phe Thr Gly Ala Gln Gly Ser
                245                 250                 255
Asn His Tyr His His Phe Val Asn Asn Ser Gly Met Ser Ala Leu Asp
            260                 265                 270
Phe Arg Tyr Ala Gln Val Ala Gln Asp Val Leu Arg Asn Gln Lys Gly
        275                 280                 285
Thr Met His Asp Ile Tyr Asp Met Leu Ala Ser Thr Gln Leu Asp Tyr
    290                 295                 300
Glu Arg Pro Gln Asp Gln Val Thr Phe Ile Asp Asn His Asp Ile Asp
305                 310                 315                 320
Arg Phe Thr Val Glu Gly Arg Asp Thr Arg Thr Thr Asp Ile Gly Leu
                325                 330                 335
Ala Phe Leu Leu Thr Ser Arg Gly Val Pro Ala Ile Tyr Tyr Gly Thr
            340                 345                 350
Glu Asn Tyr Met Thr Gly Lys Gly Asp Pro Gly Asn Arg Lys Met Met
        355                 360                 365
Glu Ser Phe Asp Gln Thr Thr Thr Ala Tyr Gln Val Ile Gln Lys Leu
    370                 375                 380
Ala Pro Leu Arg Gln Glu Asn Lys Ala Val Val Tyr Gly Ser Thr Lys
385                 390                 395                 400
Glu Arg Trp Ile Asn Asp Asp Val Leu Ile Tyr Glu Arg Ser Phe Asn
                405                 410                 415
Gly Asp Tyr Leu Leu Val Ala Ile Asn Lys Asn Val Asn Gln Ala Tyr
            420                 425                 430
Thr Ile Ser Gly Leu Leu Thr Glu Met Pro Ala Gln Val Tyr His Asp
        435                 440                 445
Val Leu Asp Ser Leu Leu Asp Gly Gln Ser Leu Ala Val Lys Glu Asn

450                 455                 460
Gly Thr Val Asp Ser Phe Leu Leu Gly Pro Gly Glu Val Ser Val Trp
465                 470                 475                 480
Gln His Ile Ser Glu Ser Gly Ser Ala Pro Val Ile Gly Gln Val Gly
                485                 490                 495
Pro Pro Met Gly Lys Pro Gly Asp Ala Val Lys Ile Ser Gly Ser Gly
            500                 505                 510
Phe Gly Ser Glu Pro Gly Thr Val Tyr Phe Arg Asp Thr Lys Ile Asp
        515                 520                 525
Val Leu Thr Trp Asp Asp Glu Thr Ile Val Ile Thr Leu Pro Glu Thr
    530                 535                 540
Leu Gly Gly Lys Ala Gln Ile Ser Val Thr Asn Ser Asp Gly Val Thr
545                 550                 555                 560
Ser Asn Gly Tyr Asp Phe Gln Leu Leu Thr Gly Lys Gln Glu Ser Val
                565                 570                 575
Arg Phe Val Val Asp Asn Ala His Thr Asn Tyr Gly Glu Asn Val Tyr
            580                 585                 590
Leu Val Gly Asn Val Pro Glu Leu Gly Asn Trp Asn Pro Ala Asp Ala
        595                 600                 605
Ile Gly Pro Met Phe Asn Gln Val Val Tyr Ser Tyr Pro Thr Trp Tyr
    610                 615                 620
Tyr Asp Val Ser Val Pro Ala Asp Thr Ala Leu Glu Phe Lys Phe Ile
625                 630                 635                 640
Ile Val Asp Gly Asn Gly Asn Val Thr Trp Glu Ser Gly Gly Asn His
                645                 650                 655
Asn Tyr Arg Val Thr Ser Gly Ser Thr Asp Thr Val Arg Val Ser Phe
            660                 665                 670
Arg Arg

<210>  2
<211>  2022
<212>  DNA
<213>  Bacillus clarkii 7364
<400>  2
agt aat gca acg aac gat ttg tcg aat gtc aat tat gcg gag gaa gtc      48
Ser Asn Ala Thr Asn Asp Leu Ser Asn Val Asn Tyr Ala Glu Glu Val
1               5                   10                  15

att tat cac att gta aca gat cgg ttt aaa gac gga gat cct gac aac      96
Ile Tyr His Ile Val Thr Asp Arg Phe Lys Asp Gly Asp Pro Asp Asn
            20                  25                  30

aat cct caa gga cag ctg ttt agt aat ggt tgc agt gat ctc aca aag      144
Asn Pro Gln Gly Gln Leu Phe Ser Asn Gly Cys Ser Asp Leu Thr Lys
        35                  40                  45

tat tgc ggt ggt gac tgg cag ggc att atc gat gaa att gaa agc ggt      192
Tyr Cys Gly Gly Asp Trp Gln Gly Ile Ile Asp Glu Ile Glu Ser Gly
    50                  55                  60

tac cta ccg gat atg gga att act gct ctg tgg atc tcc cct cct gtt      240
Tyr Leu Pro Asp Met Gly Ile Thr Ala Leu Trp Ile Ser Pro Pro Val
65                  70                  75                  80

gag aat gta ttt gat tta cat cct gaa ggc ttt tcc tct tat cac ggg      288
Glu Asn Val Phe Asp Leu His Pro Glu Gly Phe Ser Ser Tyr His Gly
                85                  90                  95

tat tgg gcc cga gac ttt aaa aag aca aac cct ttc ttc gga gat ttt      336
Tyr Trp Ala Arg Asp Phe Lys Lys Thr Asn Pro Phe Phe Gly Asp Phe
            100                 105                 110

gat gat ttt tcc cga cta atc gaa aca gct cat gca cat gac ata aaa      384
Asp Asp Phe Ser Arg Leu Ile Glu Thr Ala His Ala His Asp Ile Lys
        115                 120                 125

gta gtt att gat ttt gta cct aac cat act tcc cct gta gac atc gag      432
Val Val Ile Asp Phe Val Pro Asn His Thr Ser Pro Val Asp Ile Glu
    130                 135                 140

gat ggt gca ttg tat gac aac ggt aca tta ctg ggc cac tat tca acg 480
Asp Gly Ala Leu Tyr Asp Asn Gly Thr Leu Leu Gly His Tyr Ser Thr
145                 150                 155                 160

gat gca aac aat tat ttt tat aac tat ggt ggt tca gac ttc tca gac 528
Asp Ala Asn Asn Tyr Phe Tyr Asn Tyr Gly Gly Ser Asp Phe Ser Asp
                165                 170                 175

tat gaa aat agc atc tat cga aac ttg tat gat tta gca agt ctt aac 576
Tyr Glu Asn Ser Ile Tyr Arg Asn Leu Tyr Asp Leu Ala Ser Leu Asn
            180                 185                 190

cag caa cat tcc ttt att gat aaa tac tta aaa gaa tct att caa tta 624
Gln Gln His Ser Phe Ile Asp Lys Tyr Leu Lys Glu Ser Ile Gln Leu
        195                 200                 205

tgg ttg gat acg gga att gac ggg att cgc gtg gat gcg gtt gca cac 672
Trp Leu Asp Thr Gly Ile Asp Gly Ile Arg Val Asp Ala Val Ala His
    210                 215                 220

atg cct ttg ggc tgg caa aaa gca ttt atc tca tct gtc tat gat tac 720
Met Pro Leu Gly Trp Gln Lys Ala Phe Ile Ser Ser Val Tyr Asp Tyr
225                 230                 235                 240

aat cca gtt ttt acc ttt ggt gaa tgg ttt aca gga gca caa ggc agc 768
Asn Pro Val Phe Thr Phe Gly Glu Trp Phe Thr Gly Ala Gln Gly Ser
                245                 250                 255

aat cat tac cac cat ttt gtc aac aac agt ggc atg agc gcc ctt gat 816
Asn His Tyr His His Phe Val Asn Asn Ser Gly Met Ser Ala Leu Asp
            260                 265                 270

ttt cgc tat gct cag gta gcg cag gat gta tta aga aat caa aag gga 864
Phe Arg Tyr Ala Gln Val Ala Gln Asp Val Leu Arg Asn Gln Lys Gly
        275                 280                 285

acg atg cat gac att tac gac atg ttg gca agc act caa tta gat tat 912
Thr Met His Asp Ile Tyr Asp Met Leu Ala Ser Thr Gln Leu Asp Tyr
    290                 295                 300

gag cgg ccg caa gat caa gta acc ttt att gat aat cat gat atc gat 960
Glu Arg Pro Gln Asp Gln Val Thr Phe Ile Asp Asn His Asp Ile Asp
305                 310                 315                 320

cgc ttt acg gtg gaa ggc cga gat aca agg aca acg gac atc gga ctg 1008
Arg Phe Thr Val Glu Gly Arg Asp Thr Arg Thr Thr Asp Ile Gly Leu
                325                 330                 335

gca ttt ctt ttg aca tca aga ggc gta ccg gct att tat tat ggt acg 1056
Ala Phe Leu Leu Thr Ser Arg Gly Val Pro Ala Ile Tyr Tyr Gly Thr
            340                 345                 350

gaa aac tat atg act ggt aaa gga gat cca gga aac aga aaa atg atg 1104
Glu Asn Tyr Met Thr Gly Lys Gly Asp Pro Gly Asn Arg Lys Met Met
        355                 360                 365

gag agc ttt gat caa aca acg aca gcc tat cag gtc atc caa aag ctg 1152
Glu Ser Phe Asp Gln Thr Thr Thr Ala Tyr Gln Val Ile Gln Lys Leu
    370                 375                 380

gca ccg ctc cga caa gaa aat aaa gcg gtg gta tat ggt tca aca aaa 1200
Ala Pro Leu Arg Gln Glu Asn Lys Ala Val Val Tyr Gly Ser Thr Lys
385                 390                 395                 400

gaa cgt tgg att aac gat gat gtg ctc att tat gaa cga tcg ttt aat 1248
Glu Arg Trp Ile Asn Asp Asp Val Leu Ile Tyr Glu Arg Ser Phe Asn
                405                 410                 415

gga gat tat ctt tta gtc gca att aat aaa aat gta aat caa gct tat 1296
Gly Asp Tyr Leu Leu Val Ala Ile Asn Lys Asn Val Asn Gln Ala Tyr
            420                 425                 430

act att tcc ggt ttg ctc acg gaa atg ccc gcg caa gtc tat cat gat 1344
Thr Ile Ser Gly Leu Leu Thr Glu Met Pro Ala Gln Val Tyr His Asp
        435                 440                 445

gtt tta gac agc tta tta gac ggt caa tcg tta gca gta aaa gaa aat 1392
Val Leu Asp Ser Leu Leu Asp Gly Gln Ser Leu Ala Val Lys Glu Asn
    450                 455                 460

ggt aca gtt gat tcc ttt ctg cta gga cca ggt gaa gta agt gta tgg 1440
Gly Thr Val Asp Ser Phe Leu Leu Gly Pro Gly Glu Val Ser Val Trp
465                 470                 475                 480

cag cat ata agt gaa agt ggt tcc gct cct gtt att ggt caa gta ggc 1488
Gln His Ile Ser Glu Ser Gly Ser Ala Pro Val Ile Gly Gln Val Gly
                485                 490                 495

ccg cct atg ggg aaa cct gga gat gct gtg aag att agt ggc agc gga 1536
Pro Pro Met Gly Lys Pro Gly Asp Ala Val Lys Ile Ser Gly Ser Gly
            500                 505                 510

ttt ggt tct gag cct ggc acc gtg tac ttc aga gat acg aaa ata gac 1584
Phe Gly Ser Glu Pro Gly Thr Val Tyr Phe Arg Asp Thr Lys Ile Asp
        515                 520                 525

gtg tta act tgg gat gat gaa acg att gtg atc aca ctg ccg gaa aca 1632
Val Leu Thr Trp Asp Asp Glu Thr Ile Val Ile Thr Leu Pro Glu Thr
    530                 535                 540

tta gga gga aaa gcg caa atc agt gtt act aac tct gac ggc gtg aca 1680
Leu Gly Gly Lys Ala Gln Ile Ser Val Thr Asn Ser Asp Gly Val Thr
545                 550                 555                 560

agt aac ggc tat gat ttt cag ttg ttg aca ggt aag cag gaa tct gtt 1728
Ser Asn Gly Tyr Asp Phe Gln Leu Leu Thr Gly Lys Gln Glu Ser Val
                565                 570                 575

cgt ttc gtt gtg gat aat gcg cat acc aat tat ggg gaa aat gtt tat 1776
Arg Phe Val Val Asp Asn Ala His Thr Asn Tyr Gly Glu Asn Val Tyr
            580                 585                 590

ctt gtt gga aat gtt cct gag ctt ggg aat tgg aac cct gcc gac gca 1824
Leu Val Gly Asn Val Pro Glu Leu Gly Asn Trp Asn Pro Ala Asp Ala
        595                 600                 605

atc gga cca atg ttt aat caa gtc gtt tat tcc tat cca acc tgg tat 1872
Ile Gly Pro Met Phe Asn Gln Val Val Tyr Ser Tyr Pro Thr Trp Tyr
    610                 615                 620

tac gat gtc agt gtt ccc gcg gat acc gcg ttg gaa ttt aag ttt att 1920
Tyr Asp Val Ser Val Pro Ala Asp Thr Ala Leu Glu Phe Lys Phe Ile
625                 630                 635                 640

att gtc gat gga aat gga aat gtt act tgg gaa agc ggg ggt aat cac 1968
Ile Val Asp Gly Asn Gly Asn Val Thr Trp Glu Ser Gly Gly Asn His
                645                 650                 655

aat tat cgt gtt acc tcg gga agc acg gat act gtt cgt gta agt ttt 2016
Asn Tyr Arg Val Thr Ser Gly Ser Thr Asp Thr Val Arg Val Ser Phe
            660                 665                 670

cga agg 2022
Arg Arg

<210> 3
<211> 32

<212>  DNA
<213>  Artificial Sequence
<220>
<223>  Primer
<400>  3
aaagcatgcc attatgagga ggatttaacg tg  32

<210>  4
<211>  33
<212>  DNA
<213>  Artificial Sequence
<220>
<223>  Primer
<400>  4
aaagagctct gcatcggctc tactcatttc atc  33

<210>  5
<211>  34
<212>  DNA
<213>  Artificial Sequence
<220>
<223>  Primer
<400>  5
ggaattactg ctctggcgat ctcccctcct gttg  34

<210>  6
<211>  34
<212>  DNA
<213>  Artificial Sequence
<220>
<223>  Primer
<400>  6
ggaattactg ctctgtgtat ctcccctcct gttg  34

<210>  7
<211>  34
<212>  DNA
<213>  Artificial Sequence
<220>
<223>  Primer
<400>  7
ggaattactg ctctggagat ctcccctcct gttg  34

<210>  8
<211>  34
<212>  DNA
<213>  Artificial Sequence
<220>
<223>  Primer
<400>  8
ggaattactg ctctgtttat ctcccctcct gttg  34

<210>  9
<211>  34
<212>  DNA
<213>  Artificial Sequence
<220>
<223>  Primer
<400>  9
ggaattactg ctctgcacat ctcccctcct gttg  34

<210>  10
<211>  34
<212>  DNA
<213>  Artificial Sequence
<220>
<223>  Primer
<400>  10
ggaattactg ctctgataat ctcccctcct gttg  34

<210>  11
<211>  34
<212>  DNA
<213>  Artificial Sequence
<220>
<223>  Primer
<400>  11
ggaattactg ctctgaagat ctcccctcct gttg  34

<210>  12
<211>  34
<212>  DNA
<213>  Artificial Sequence
<220>
<223>  Primer
<400>  12
ggaattactg ctctgatgat ctcccctcct gttg  34

<210>  13
<211>  34
<212>  DNA
<213>  Artificial Sequence
<220>
<223>  Primer
<400>  13
ggaattactg ctctgcagat ctcccctcct gttg  34

<210>  14
<211>  34
<212>  DNA
<213>  Artificial Sequence
<220>
<223>  Primer
<400>  14
ggaattactg ctctgtcgat ctcccctcct gttg  34

<210>  15
<211>  34
<212>  DNA
<213>  Artificial Sequence
<220>
<223>  Primer
<400>  15
ggaattactg ctctgacgat ctcccctcct gttg  34

<210>  16
<211>  34
<212>  DNA
<213>  Artificial Sequence
<220>
<223>  Primer
<400>  16
ggaattactg ctctggtgat ctcccctcct gttg  34

<210>  17
<211>  34
<212>  DNA
<213>  Artificial Sequence
<220>
<223>  Primer
<400>  17
ggaattactg ctctgtacat ctcccctcct gttg  34

<210>  18
<211>  44
<212>  DNA
<213>  Artificial Sequence
<220>

<223>  Primer
<400>  18
cataaaagta gttattgatt ttccacctaa ccatacttcc cctg  44

<210>  19
<211>  44
<212>  DNA
<213>  Artificial Sequence
<220>
<223>  Primer
<400>  19
cataaaagta gttattgatt tttcacctaa ccatacttcc cctg  44

<210>  20
<211>  44
<212>  DNA
<213>  Artificial Sequence
<220>
<223>  Primer
<400>  20
cataaaagta gttattgatt ttacacctaa ccatacttcc cctg  44

<210>  21
<211>  33
<212>  DNA
<213>  Artificial Sequence
<220>
<223>  Primer
<400>  21
cgcgtggatg cggttcacca catgcctttg ggc  33

<210>  22
<211>  33
<212>  DNA
<213>  Artificial Sequence
<220>
<223>  Primer
<400>  22
cgcgtggatg cggttaaaca catgcctttg ggc  33

<210>  23
<211>  33
<212>  DNA
<213>  Artificial Sequence
<220>
<223>  Primer
<400>  23
cgcgtggatg cggttcgaca catgcctttg ggc  33

<210>  24
<211>  36
<212>  DNA
<213>  Artificial Sequence
<220>
<223>  Primer
<400>  24
catcaagagg cgtaccgtgt atttattatg gtacgg  36

<210>  25
<211>  36
<212>  DNA
<213>  Artificial Sequence
<220>
<223>  Primer
<400>  25
catcaagagg cgtaccggat atttattatg gtacgg  36

<210>  26
<211>  36

<212>  DNA
<213>  Artificial Sequence
<220>
<223>  Primer
<400>  26
catcaagagg cgtaccggag atttattatg gtacgg  36

<210>  27
<211>  36
<212>  DNA
<213>  Artificial Sequence
<220>
<223>  Primer
<400>  27
catcaagagg cgtaccgcat atttattatg gtacgg  36

<210>  28
<211>  36
<212>  DNA
<213>  Artificial Sequence
<220>
<223>  Primer
<400>  28
catcaagagg cgtaccgatt atttattatg gtacgg  36

<210>  29
<211>  36
<212>  DNA
<213>  Artificial Sequence
<220>
<223>  Primer
<400>  29
catcaagagg cgtaccgctt atttattatg gtacgg  36

<210>  30
<211>  36
<212>  DNA
<213>  Artificial Sequence
<220>
<223>  Primer
<400>  30
catcaagagg cgtaccgaat atttattatg gtacgg  36

<210>  31
<211>  36
<212>  DNA
<213>  Artificial Sequence
<220>
<223>  Primer
<400>  31
catcaagagg cgtaccgtct atttattatg gtacgg  36

<210>  32
<211>  36
<212>  DNA
<213>  Artificial Sequence
<220>
<223>  Primer
<400>  32
catcaagagg cgtaccgact atttattatg gtacgg  36

<210>  33
<211>  36
<212>  DNA
<213>  Artificial Sequence
<220>
<223>  Primer
<400>  33
catcaagagg cgtaccgtat atttattatg gtacgg  36

<210>  34
<211>  40
<212>  DNA
<213>  Artificial Sequence
<220>
<223>  Primer
<400>  34
ctatatgact ggtaagggag caccaggaaa cagaaaaatg  40

<210>  35
<211>  40
<212>  DNA
<213>  Artificial Sequence
<220>
<223>  Primer
<400>  35
ctatatgact ggtaagggat gcccaggaaa cagaaaatg  40

<210>  36
<211>  40
<212>  DNA
<213>  Artificial Sequence
<220>
<223>  Primer
<400>  36
ctatatgact ggtaagggag agccaggaaa cagaaaatg  40

<210>  37
<211>  40
<212>  DNA
<213>  Artificial Sequence
<220>
<223>  Primer
<400>  37
ctatatgact ggtaagggat ttccaggaaa cagaaaatg  40

<210>  38
<211>  40
<212>  DNA
<213>  Artificial Sequence
<220>
<223>  Primer
<400>  38
ctatatgact ggtaagggac acccaggaaa cagaaaatg  40

<210>  39
<211>  40
<212>  DNA
<213>  Artificial Sequence
<220>
<223>  Primer
<400>  39
ctatatgact ggtaagggaa tcccaggaaa cagaaaatg  40

<210>  40
<211>  40
<212>  DNA
<213>  Artificial Sequence
<220>
<223>  Primer
<400>  40
ctatatgact ggtaagggaa aaccaggaaa cagaaaatg  40

<210>  41
<211>  40
<212>  DNA
<213>  Artificial Sequence
<220>

<223>  Primer
<400>  41
ctatatgact ggtaagggac tgccaggaaa cagaaaaatg  40

<210>  42
<211>  40
<212>  DNA
<213>  Artificial Sequence
<220>
<223>  Primer
<400>  42
ctatatgact ggtaagggaa tgccaggaaa cagaaaaatg  40

<210>  43
<211>  40
<212>  DNA
<213>  Artificial Sequence
<220>
<223>  Primer
<400>  43
ctatatgact ggtaagggaa acccaggaaa cagaaaaatg  40

<210>  44
<211>  40
<212>  DNA
<213>  Artificial Sequence
<220>
<223>  Primer
<400>  44
ctatatgact ggtaagggac ccccaggaaa cagaaaaatg  40

<210>  45
<211>  40
<212>  DNA
<213>  Artificial Sequence
<220>
<223>  Primer
<400>  45
ctatatgact ggtaagggac aaccaggaaa cagaaaaatg  40

<210>  46
<211>  40
<212>  DNA
<213>  Artificial Sequence
<220>
<223>  Primer
<400>  46
ctatatgact ggtaagggaa gcccaggaaa cagaaaaatg  40

<210>  47
<211>  40
<212>  DNA
<213>  Artificial Sequence
<220>
<223>  Primer
<400>  47
ctatatgact ggtaagggaa ccccaggaaa cagaaaaatg  40

<210>  48
<211>  40
<212>  DNA
<213>  Artificial Sequence
<220>
<223>  Primer
<400>  48
ctatatgact ggtaagggag tgccaggaaa cagaaaaatg  40

<210>  49
<211>  40

<212>  DNA
<213>  Artificial Sequence
<220>
<223>  Primer
<400>  49
ctatatgact ggtaagggat ggccaggaaa cagaaaaatg  40

<210>  50
<211>  40
<212>  DNA
<213>  Artificial Sequence
<220>
<223>  Primer
<400>  50
ctatatgact ggtaagggat atccaggaaa cagaaaaatg  40

SEQUENCE LISTING
<110> Nihon Shokuhin Kako Co., Ltd. ; Japan Agency for Marine-Earth Science and Technology
<120>ƒÁ-Cyclodextrin glucanotransferase mutants and genes coding the same
<130>  A75107H
<160>  50

<210>  1
<211>  674
<212>  PRT
<213>  Bacillus clarkii 7364
<400>  1
Ser Asn Ala Thr Asn Asp Leu Ser Asn Val Asn Tyr Ala Glu Glu Val
1               5                   10                  15
Ile Tyr His Ile Val Thr Asp Arg Phe Lys Asp Gly Asp Pro Asp Asn
            20                  25                  30
Asn Pro Gln Gly Gln Leu Phe Ser Asn Gly Cys Ser Asp Leu Thr Lys
        35                  40                  45
Tyr Cys Gly Gly Asp Trp Gln Gly Ile Ile Asp Glu Ile Glu Ser Gly
    50                  55                  60
Tyr Leu Pro Asp Met Gly Ile Thr Ala Leu Trp Ile Ser Pro Pro Val
65                  70                  75                  80
Glu Asn Val Phe Asp Leu His Pro Glu Gly Phe Ser Ser Tyr His Gly
                85                  90                  95
Tyr Trp Ala Arg Asp Phe Lys Lys Thr Asn Pro Phe Phe Gly Asp Phe
            100                 105                 110
Asp Asp Phe Ser Arg Leu Ile Glu Thr Ala His Ala His Asp Ile Lys
        115                 120                 125
Val Val Ile Asp Phe Val Pro Asn His Thr Ser Pro Val Asp Ile Glu
    130                 135                 140
Asp Gly Ala Leu Tyr Asp Asn Gly Thr Leu Leu Gly His Tyr Ser Thr
145                 150                 155                 160
Asp Ala Asn Asn Tyr Phe Tyr Asn Tyr Gly Gly Ser Asp Phe Ser Asp
                165                 170                 175
Tyr Glu Asn Ser Ile Tyr Arg Asn Leu Tyr Asp Leu Ala Ser Leu Asn
            180                 185                 190
Gln Gln His Ser Phe Ile Asp Lys Tyr Leu Lys Glu Ser Ile Gln Leu
        195                 200                 205
Trp Leu Asp Thr Gly Ile Asp Gly Ile Arg Val Asp Ala Val Ala His
    210                 215                 220
Met Pro Leu Gly Trp Gln Lys Ala Phe Ile Ser Ser Val Tyr Asp Tyr
225                 230                 235                 240
Asn Pro Val Phe Thr Phe Gly Glu Trp Phe Thr Gly Ala Gln Gly Ser
                245                 250                 255
Asn His Tyr His His Phe Val Asn Asn Ser Gly Met Ser Ala Leu Asp
            260                 265                 270
Phe Arg Tyr Ala Gln Val Ala Gln Asp Val Leu Arg Asn Gln Lys Gly
        275                 280                 285
Thr Met His Asp Ile Tyr Asp Met Leu Ala Ser Thr Gln Leu Asp Tyr
    290                 295                 300
Glu Arg Pro Gln Asp Gln Val Thr Phe Ile Asp Asn His Asp Ile Asp
305                 310                 315                 320
Arg Phe Thr Val Glu Gly Arg Asp Thr Arg Thr Thr Asp Ile Gly Leu
                325                 330                 335
Ala Phe Leu Leu Thr Ser Arg Gly Val Pro Ala Ile Tyr Tyr Gly Thr
            340                 345                 350
Glu Asn Tyr Met Thr Gly Lys Gly Asp Pro Gly Asn Arg Lys Met Met
        355                 360                 365
Glu Ser Phe Asp Gln Thr Thr Thr Ala Tyr Gln Val Ile Gln Lys Leu
    370                 375                 380
Ala Pro Leu Arg Gln Glu Asn Lys Ala Val Val Tyr Gly Ser Thr Lys
385                 390                 395                 400
Glu Arg Trp Ile Asn Asp Asp Val Leu Ile Tyr Glu Arg Ser Phe Asn
                405                 410                 415
Gly Asp Tyr Leu Leu Val Ala Ile Asn Lys Asn Val Asn Gln Ala Tyr
            420                 425                 430
Thr Ile Ser Gly Leu Leu Thr Glu Met Pro Ala Gln Val Tyr His Asp
        435                 440                 445
Val Leu Asp Ser Leu Leu Asp Gly Gln Ser Leu Ala Val Lys Glu Asn

450                 455                 460
Gly Thr Val Asp Ser Phe Leu Leu Gly Pro Gly Glu Val Ser Val Trp
465                 470                 475                 480
Gln His Ile Ser Glu Ser Gly Ser Ala Pro Val Ile Gly Gln Val Gly
                485                 490                 495
Pro Pro Met Gly Lys Pro Gly Asp Ala Val Lys Ile Ser Gly Ser Gly
            500                 505                 510
Phe Gly Ser Glu Pro Gly Thr Val Tyr Phe Arg Asp Thr Lys Ile Asp
        515                 520                 525
Val Leu Thr Trp Asp Asp Glu Thr Ile Val Ile Thr Leu Pro Glu Thr
    530                 535                 540
Leu Gly Gly Lys Ala Gln Ile Ser Val Thr Asn Ser Asp Gly Val Thr
545                 550                 555                 560
Ser Asn Gly Tyr Asp Phe Gln Leu Leu Thr Gly Lys Gln Glu Ser Val
                565                 570                 575
Arg Phe Val Val Asp Asn Ala His Thr Asn Tyr Gly Glu Asn Val Tyr
            580                 585                 590
Leu Val Gly Asn Val Pro Glu Leu Gly Asn Trp Asn Pro Ala Asp Ala
        595                 600                 605
Ile Gly Pro Met Phe Asn Gln Val Val Tyr Ser Tyr Pro Thr Trp Tyr
    610                 615                 620
Tyr Asp Val Ser Val Pro Ala Asp Thr Ala Leu Glu Phe Lys Phe Ile
625                 630                 635                 640
Ile Val Asp Gly Asn Gly Asn Val Thr Trp Glu Ser Gly Gly Asn His
                645                 650                 655
Asn Tyr Arg Val Thr Ser Gly Ser Thr Asp Thr Val Arg Val Ser Phe
            660                 665                 670
Arg Arg

<210>  2
<211>  2022
<212>  DNA
<213>  Bacillus clarkii 7364
<400>  2
agt aat gca acg aac gat ttg tcg aat gtc aat tat gcg gag gaa gtc      48
Ser Asn Ala Thr Asn Asp Leu Ser Asn Val Asn Tyr Ala Glu Glu Val
1               5                   10                  15

att tat cac att gta aca gat cgg ttt aaa gac gga gat cct gac aac      96
Ile Tyr His Ile Val Thr Asp Arg Phe Lys Asp Gly Asp Pro Asp Asn
            20                  25                  30

aat cct caa gga cag ctg ttt agt aat ggt tgc agt gat ctc aca aag      144
Asn Pro Gln Gly Gln Leu Phe Ser Asn Gly Cys Ser Asp Leu Thr Lys
        35                  40                  45

tat tgc ggt ggt gac tgg cag ggc att atc gat gaa att gaa agc ggt      192
Tyr Cys Gly Gly Asp Trp Gln Gly Ile Ile Asp Glu Ile Glu Ser Gly
    50                  55                  60

tac cta ccg gat atg gga att act gct ctg tgg atc tcc cct cct gtt      240
Tyr Leu Pro Asp Met Gly Ile Thr Ala Leu Trp Ile Ser Pro Pro Val
65                  70                  75                  80

gag aat gta ttt gat tta cat cct gaa ggc ttt tcc tct tat cac ggg      288
Glu Asn Val Phe Asp Leu His Pro Glu Gly Phe Ser Ser Tyr His Gly
                85                  90                  95

tat tgg gcc cga gac ttt aaa aag aca aac cct ttc ttc gga gat ttt      336
Tyr Trp Ala Arg Asp Phe Lys Lys Thr Asn Pro Phe Phe Gly Asp Phe
            100                 105                 110

gat gat ttt tcc cga cta atc gaa aca gct cat gca cat gac ata aaa      384
Asp Asp Phe Ser Arg Leu Ile Glu Thr Ala His Ala His Asp Ile Lys
        115                 120                 125

gta gtt att gat ttt gta cct aac cat act tcc cct gta gac atc gag      432
Val Val Ile Asp Phe Val Pro Asn His Thr Ser Pro Val Asp Ile Glu
    130                 135                 140

gat ggt gca ttg tat gac aac ggt aca tta ctg ggc cac tat tca acg 480
Asp Gly Ala Leu Tyr Asp Asn Gly Thr Leu Leu Gly His Tyr Ser Thr
145                 150                 155                 160

gat gca aac aat tat ttt tat aac tat ggt ggt tca gac ttc tca gac 528
Asp Ala Asn Asn Tyr Phe Tyr Asn Tyr Gly Gly Ser Asp Phe Ser Asp
                165                 170                 175

tat gaa aat agc atc tat cga aac ttg tat gat tta gca agt ctt aac 576
Tyr Glu Asn Ser Ile Tyr Arg Asn Leu Tyr Asp Leu Ala Ser Leu Asn
            180                 185                 190

cag caa cat tcc ttt att gat aaa tac tta aaa gaa tct att caa tta 624
Gln Gln His Ser Phe Ile Asp Lys Tyr Leu Lys Glu Ser Ile Gln Leu
        195                 200                 205

tgg ttg gat acg gga att gac ggg att cgc gtg gat gcg gtt gca cac 672
Trp Leu Asp Thr Gly Ile Asp Gly Ile Arg Val Asp Ala Val Ala His
    210                 215                 220

atg cct ttg ggc tgg caa aaa gca ttt atc tca tct gtc tat gat tac 720
Met Pro Leu Gly Trp Gln Lys Ala Phe Ile Ser Ser Val Tyr Asp Tyr
225                 230                 235                 240

aat cca gtt ttt acc ttt ggt gaa tgg ttt aca gga gca caa ggc agc 768
Asn Pro Val Phe Thr Phe Gly Glu Trp Phe Thr Gly Ala Gln Gly Ser
                245                 250                 255

aat cat tac cac cat ttt gtc aac aac agt ggc atg agc gcc ctt gat 816
Asn His Tyr His His Phe Val Asn Asn Ser Gly Met Ser Ala Leu Asp
            260                 265                 270

ttt cgc tat gct cag gta gcg cag gat gta tta aga aat caa aag gga 864
Phe Arg Tyr Ala Gln Val Ala Gln Asp Val Leu Arg Asn Gln Lys Gly
        275                 280                 285

acg atg cat gac att tac gac atg ttg gca agc act caa tta gat tat 912
Thr Met His Asp Ile Tyr Asp Met Leu Ala Ser Thr Gln Leu Asp Tyr
    290                 295                 300

gag cgg ccg caa gat caa gta acc ttt att gat aat cat gat atc gat 960
Glu Arg Pro Gln Asp Gln Val Thr Phe Ile Asp Asn His Asp Ile Asp
305                 310                 315                 320

cgc ttt acg gtg gaa ggc cga gat aca agg aca acg gac atc gga ctg 1008
Arg Phe Thr Val Glu Gly Arg Asp Thr Arg Thr Thr Asp Ile Gly Leu
                325                 330                 335

gca ttt ctt ttg aca tca aga ggc gta ccg gct att tat tat ggt acg 1056
Ala Phe Leu Leu Thr Ser Arg Gly Val Pro Ala Ile Tyr Tyr Gly Thr
            340                 345                 350

gaa aac tat atg act ggt aaa gga gat cca gga aac aga aaa atg atg 1104
Glu Asn Tyr Met Thr Gly Lys Gly Asp Pro Gly Asn Arg Lys Met Met
        355                 360                 365

gag agc ttt gat caa aca acg aca gcc tat cag gtc atc caa aag ctg 1152
Glu Ser Phe Asp Gln Thr Thr Thr Ala Tyr Gln Val Ile Gln Lys Leu
    370                 375                 380

gca ccg ctc cga caa gaa aat aaa gcg gtg gta tat ggt tca aca aaa 1200
Ala Pro Leu Arg Gln Glu Asn Lys Ala Val Val Tyr Gly Ser Thr Lys
385                 390                 395                 400

gaa cgt tgg att aac gat gat gtg ctc att tat gaa cga tcg ttt aat 1248
Glu Arg Trp Ile Asn Asp Asp Val Leu Ile Tyr Glu Arg Ser Phe Asn
                405                 410                 415

gga gat tat ctt tta gtc gca att aat aaa aat gta aat caa gct tat          1296
Gly Asp Tyr Leu Leu Val Ala Ile Asn Lys Asn Val Asn Gln Ala Tyr
            420                 425                 430

act att tcc ggt ttg ctc acg gaa atg ccc gcg caa gtc tat cat gat          1344
Thr Ile Ser Gly Leu Leu Thr Glu Met Pro Ala Gln Val Tyr His Asp
        435                 440                 445

gtt tta gac agc tta tta gac ggt caa tcg tta gca gta aaa gaa aat          1392
Val Leu Asp Ser Leu Leu Asp Gly Gln Ser Leu Ala Val Lys Glu Asn
    450                 455                 460

ggt aca gtt gat tcc ttt ctg cta gga cca ggt gaa gta agt gta tgg          1440
Gly Thr Val Asp Ser Phe Leu Leu Gly Pro Gly Glu Val Ser Val Trp
465                 470                 475                 480

cag cat ata agt gaa agt ggt tcc gct cct gtt att ggt caa gta ggc          1488
Gln His Ile Ser Glu Ser Gly Ser Ala Pro Val Ile Gly Gln Val Gly
                485                 490                 495

ccg cct atg ggg aaa cct gga gat gct gtg aag att agt ggc agc gga          1536
Pro Pro Met Gly Lys Pro Gly Asp Ala Val Lys Ile Ser Gly Ser Gly
            500                 505                 510

ttt ggt tct gag cct ggc acc gtg tac ttc aga gat acg aaa ata gac          1584
Phe Gly Ser Glu Pro Gly Thr Val Tyr Phe Arg Asp Thr Lys Ile Asp
        515                 520                 525

gtg tta act tgg gat gat gaa acg att gtg atc aca ctg ccg gaa aca          1632
Val Leu Thr Trp Asp Asp Glu Thr Ile Val Ile Thr Leu Pro Glu Thr
    530                 535                 540

tta gga gga aaa gcg caa atc agt gtt act aac tct gac ggc gtg aca          1680
Leu Gly Gly Lys Ala Gln Ile Ser Val Thr Asn Ser Asp Gly Val Thr
545                 550                 555                 560

agt aac ggc tat gat ttt cag ttg ttg aca ggt aag cag gaa tct gtt          1728
Ser Asn Gly Tyr Asp Phe Gln Leu Leu Thr Gly Lys Gln Glu Ser Val
                565                 570                 575

cgt ttc gtt gtg gat aat gcg cat acc aat tat ggg gaa aat gtt tat          1776
Arg Phe Val Val Asp Asn Ala His Thr Asn Tyr Gly Glu Asn Val Tyr
            580                 585                 590

ctt gtt gga aat gtt cct gag ctt ggg aat tgg aac cct gcc gac gca          1824
Leu Val Gly Asn Val Pro Glu Leu Gly Asn Trp Asn Pro Ala Asp Ala
        595                 600                 605

atc gga cca atg ttt aat caa gtc gtt tat tcc tat cca acc tgg tat          1872
Ile Gly Pro Met Phe Asn Gln Val Val Tyr Ser Tyr Pro Thr Trp Tyr
    610                 615                 620

tac gat gtc agt gtt ccc gcg gat acc gcg ttg gaa ttt aag ttt att          1920
Tyr Asp Val Ser Val Pro Ala Asp Thr Ala Leu Glu Phe Lys Phe Ile
625                 630                 635                 640

att gtc gat gga aat gga aat gtt act tgg gaa agc ggg ggt aat cac          1968
Ile Val Asp Gly Asn Gly Asn Val Thr Trp Glu Ser Gly Gly Asn His
                645                 650                 655

aat tat cgt gtt acc tcg gga agc acg gat act gtt cgt gta agt ttt          2016
Asn Tyr Arg Val Thr Ser Gly Ser Thr Asp Thr Val Arg Val Ser Phe
            660                 665                 670

cga agg                                                                  2022
Arg Arg

<210>  3
<211>  32

<212>  DNA
<213>  Artificial Sequence
<220>
<223>  Primer
<400>  3
aaagcatgcc attatgagga ggatttaacg tg  32

<210>  4
<211>  33
<212>  DNA
<213>  Artificial Sequence
<220>
<223>  Primer
<400>  4
aaagagctct gcatcggctc tactcatttc atc  33

<210>  5
<211>  34
<212>  DNA
<213>  Artificial Sequence
<220>
<223>  Primer
<400>  5
ggaattactg ctctggcgat ctcccctcct gttg  34

<210>  6
<211>  34
<212>  DNA
<213>  Artificial Sequence
<220>
<223>  Primer
<400>  6
ggaattactg ctctgtgtat ctcccctcct gttg  34

<210>  7
<211>  34
<212>  DNA
<213>  Artificial Sequence
<220>
<223>  Primer
<400>  7
ggaattactg ctctggagat ctcccctcct gttg  34

<210>  8
<211>  34
<212>  DNA
<213>  Artificial Sequence
<220>
<223>  Primer
<400>  8
ggaattactg ctctgtttat ctcccctcct gttg  34

<210>  9
<211>  34
<212>  DNA
<213>  Artificial Sequence
<220>
<223>  Primer
<400>  9
ggaattactg ctctgcacat ctcccctcct gttg  34

<210>  10
<211>  34
<212>  DNA
<213>  Artificial Sequence
<220>
<223>  Primer
<400>  10
ggaattactg ctctgataat ctcccctcct gttg  34

<210>  11
<211>  34
<212>  DNA
<213>  Artificial Sequence
<220>
<223>  Primer
<400>  11
ggaattactg ctctgaagat ctcccctcct gttg  34

<210>  12
<211>  34
<212>  DNA
<213>  Artificial Sequence
<220>
<223>  Primer
<400>  12
ggaattactg ctctgatgat ctcccctcct gttg  34

<210>  13
<211>  34
<212>  DNA
<213>  Artificial Sequence
<220>
<223>  Primer
<400>  13
ggaattactg ctctgcagat ctcccctcct gttg  34

<210>  14
<211>  34
<212>  DNA
<213>  Artificial Sequence
<220>
<223>  Primer
<400>  14
ggaattactg ctctgtcgat ctcccctcct gttg  34

<210>  15
<211>  34
<212>  DNA
<213>  Artificial Sequence
<220>
<223>  Primer
<400>  15
ggaattactg ctctgacgat ctcccctcct gttg  34

<210>  16
<211>  34
<212>  DNA
<213>  Artificial Sequence
<220>
<223>  Primer
<400>  16
ggaattactg ctctggtgat ctcccctcct gttg  34

<210>  17
<211>  34
<212>  DNA
<213>  Artificial Sequence
<220>
<223>  Primer
<400>  17
ggaattactg ctctgtacat ctcccctcct gttg  34

<210>  18
<211>  44
<212>  DNA
<213>  Artificial Sequence
<220>

<223>  Primer
<400>  18
cataaaagta gttattgatt ttccacctaa ccatacttcc cctg  44

<210>  19
<211>  44
<212>  DNA
<213>  Artificial Sequence
<220>
<223>  Primer
<400>  19
cataaaagta gttattgatt tttcacctaa ccatacttcc cctg  44

<210>  20
<211>  44
<212>  DNA
<213>  Artificial Sequence
<220>
<223>  Primer
<400>  20
cataaaagta gttattgatt ttacacctaa ccatacttcc cctg  44

<210>  21
<211>  33
<212>  DNA
<213>  Artificial Sequence
<220>
<223>  Primer
<400>  21
cgcgtggatg cggttcacca catgcctttg ggc  33

<210>  22
<211>  33
<212>  DNA
<213>  Artificial Sequence
<220>
<223>  Primer
<400>  22
cgcgtggatg cggttaaaca catgcctttg ggc  33

<210>  23
<211>  33
<212>  DNA
<213>  Artificial Sequence
<220>
<223>  Primer
<400>  23
cgcgtggatg cggttcgaca catgcctttg ggc  33

<210>  24
<211>  36
<212>  DNA
<213>  Artificial Sequence
<220>
<223>  Primer
<400>  24
catcaagagg cgtaccgtgt atttattatg gtacgg  36

<210>  25
<211>  36
<212>  DNA
<213>  Artificial Sequence
<220>
<223>  Primer
<400>  25
catcaagagg cgtaccggat atttattatg gtacgg  36

<210>  26
<211>  36

<212>  DNA
<213>  Artificial Sequence
<220>
<223>  Primer
<400>  26
catcaagagg cgtaccggag atttattatg gtacgg  36

<210>  27
<211>  36
<212>  DNA
<213>  Artificial Sequence
<220>
<223>  Primer
<400>  27
catcaagagg cgtaccgcat atttattatg gtacgg  36

<210>  28
<211>  36
<212>  DNA
<213>  Artificial Sequence
<220>
<223>  Primer
<400>  28
catcaagagg cgtaccgatt atttattatg gtacgg  36

<210>  29
<211>  36
<212>  DNA
<213>  Artificial Sequence
<220>
<223>  Primer
<400>  29
catcaagagg cgtaccgctt atttattatg gtacgg  36

<210>  30
<211>  36
<212>  DNA
<213>  Artificial Sequence
<220>
<223>  Primer
<400>  30
catcaagagg cgtaccgaat atttattatg gtacgg  36

<210>  31
<211>  36
<212>  DNA
<213>  Artificial Sequence
<220>
<223>  Primer
<400>  31
catcaagagg cgtaccgtct atttattatg gtacgg  36

<210>  32
<211>  36
<212>  DNA
<213>  Artificial Sequence
<220>
<223>  Primer
<400>  32
catcaagagg cgtaccgact atttattatg gtacgg  36

<210>  33
<211>  36
<212>  DNA
<213>  Artificial Sequence
<220>
<223>  Primer
<400>  33
catcaagagg cgtaccgtat atttattatg gtacgg  36

<210>  34
<211>  40
<212>  DNA
<213>  Artificial Sequence
<220>
<223>  Primer
<400>  34
ctatatgact ggtaagggag caccaggaaa cagaaaaatg  40

<210>  35
<211>  40
<212>  DNA
<213>  Artificial Sequence
<220>
<223>  Primer
<400>  35
ctatatgact ggtaagggat gcccaggaaa cagaaaaatg  40

<210>  36
<211>  40
<212>  DNA
<213>  Artificial Sequence
<220>
<223>  Primer
<400>  36
ctatatgact ggtaagggag agccaggaaa cagaaaaatg  40

<210>  37
<211>  40
<212>  DNA
<213>  Artificial Sequence
<220>
<223>  Primer
<400>  37
ctatatgact ggtaagggat ttccaggaaa cagaaaaatg  40

<210>  38
<211>  40
<212>  DNA
<213>  Artificial Sequence
<220>
<223>  Primer
<400>  38
ctatatgact ggtaagggac acccaggaaa cagaaaaatg  40

<210>  39
<211>  40
<212>  DNA
<213>  Artificial Sequence
<220>
<223>  Primer
<400>  39
ctatatgact ggtaagggaa tcccaggaaa cagaaaaatg  40

<210>  40
<211>  40
<212>  DNA
<213>  Artificial Sequence
<220>
<223>  Primer
<400>  40
ctatatgact ggtaagggaa aaccaggaaa cagaaaaatg  40

<210>  41
<211>  40
<212>  DNA
<213>  Artificial Sequence
<220>

<223>  Primer
<400>  41
ctatatgact ggtaagggac tgccaggaaa cagaaaaatg  40

<210>  42
<211>  40
<212>  DNA
<213>  Artificial Sequence
<220>
<223>  Primer
<400>  42
ctatatgact ggtaagggaa tgccaggaaa cagaaaaatg  40

<210>  43
<211>  40
<212>  DNA
<213>  Artificial Sequence
<220>
<223>  Primer
<400>  43
ctatatgact ggtaagggaa acccaggaaa cagaaaaatg  40

<210>  44
<211>  40
<212>  DNA
<213>  Artificial Sequence
<220>
<223>  Primer
<400>  44
ctatatgact ggtaagggac ccccaggaaa cagaaaaatg  40

<210>  45
<211>  40
<212>  DNA
<213>  Artificial Sequence
<220>
<223>  Primer
<400>  45
ctatatgact ggtaagggac aaccaggaaa cagaaaaatg  40

<210>  46
<211>  40
<212>  DNA
<213>  Artificial Sequence
<220>
<223>  Primer
<400>  46
ctatatgact ggtaagggaa gcccaggaaa cagaaaaatg  40

<210>  47
<211>  40
<212>  DNA
<213>  Artificial Sequence
<220>
<223>  Primer
<400>  47
ctatatgact ggtaagggaa ccccaggaaa cagaaaaatg  40

<210>  48
<211>  40
<212>  DNA
<213>  Artificial Sequence
<220>
<223>  Primer
<400>  48
ctatatgact ggtaagggag tgccaggaaa cagaaaaatg  40

<210>  49
<211>  40

<212>  DNA
<213>  Artificial Sequence
<220>
<223>  Primer
<400>  49
ctatatgact ggtaagggat ggccaggaaa cagaaaaatg  40

<210>  50
<211>  40
<212>  DNA
<213>  Artificial Sequence
<220>
<223>  Primer
<400>  50
ctatatgact ggtaagggat atccaggaaa cagaaaaatg  40

SEQUENCE LISTING
<110> Nihon Shokuhin Kako Co., Ltd. ; Japan Agency for Marine-Earth Science and Technology
<120>ƒÁ-Cyclodextrin glucanotransferase mutants and genes coding the same
<130> A75107H
<160> 50

<210> 1
<211> 674
<212> PRT
<213> Bacillus clarkii 7364
<400> 1
Ser Asn Ala Thr Asn Asp Leu Ser Asn Val Asn Tyr Ala Glu Glu Val
1               5                   10                  15
Ile Tyr His Ile Val Thr Asp Arg Phe Lys Asp Gly Asp Pro Asp Asn
            20                  25                  30
Asn Pro Gln Gly Gln Leu Phe Ser Asn Gly Cys Ser Asp Leu Thr Lys
        35                  40                  45
Tyr Cys Gly Gly Asp Trp Gln Gly Ile Ile Asp Glu Ile Glu Ser Gly
    50                  55                  60
Tyr Leu Pro Asp Met Gly Ile Thr Ala Leu Trp Ile Ser Pro Pro Val
65                  70                  75                  80
Glu Asn Val Phe Asp Leu His Pro Glu Gly Phe Ser Ser Tyr His Gly
                85                  90                  95
Tyr Trp Ala Arg Asp Phe Lys Lys Thr Asn Pro Phe Phe Gly Asp Phe
            100                 105                 110
Asp Asp Phe Ser Arg Leu Ile Glu Thr Ala His Ala His Asp Ile Lys
        115                 120                 125
Val Val Ile Asp Phe Val Pro Asn His Thr Ser Pro Val Asp Ile Glu
    130                 135                 140
Asp Gly Ala Leu Tyr Asp Asn Gly Thr Leu Leu Gly His Tyr Ser Thr
145                 150                 155                 160
Asp Ala Asn Asn Tyr Phe Tyr Asn Tyr Gly Gly Ser Asp Phe Ser Asp
                165                 170                 175
Tyr Glu Asn Ser Ile Tyr Arg Asn Leu Tyr Asp Leu Ala Ser Leu Asn
            180                 185                 190
Gln Gln His Ser Phe Ile Asp Lys Tyr Leu Lys Glu Ser Ile Gln Leu
        195                 200                 205
Trp Leu Asp Thr Gly Ile Asp Gly Ile Arg Val Asp Ala Val Ala His
    210                 215                 220
Met Pro Leu Gly Trp Gln Lys Ala Phe Ile Ser Ser Val Tyr Asp Tyr
225                 230                 235                 240
Asn Pro Val Phe Thr Phe Gly Glu Trp Phe Thr Gly Ala Gln Gly Ser
                245                 250                 255
Asn His Tyr His His Phe Val Asn Asn Ser Gly Met Ser Ala Leu Asp
            260                 265                 270
Phe Arg Tyr Ala Gln Val Ala Gln Asp Val Leu Arg Asn Gln Lys Gly
        275                 280                 285
Thr Met His Asp Ile Tyr Asp Met Leu Ala Ser Thr Gln Leu Asp Tyr
    290                 295                 300
Glu Arg Pro Gln Asp Gln Val Thr Phe Ile Asp Asn His Asp Ile Asp
305                 310                 315                 320
Arg Phe Thr Val Glu Gly Arg Asp Thr Arg Thr Thr Asp Ile Gly Leu
                325                 330                 335
Ala Phe Leu Leu Thr Ser Arg Gly Val Pro Ala Ile Tyr Tyr Gly Thr
            340                 345                 350
Glu Asn Tyr Met Thr Gly Lys Gly Asp Pro Gly Asn Arg Lys Met Met
        355                 360                 365
Glu Ser Phe Asp Gln Thr Thr Thr Ala Tyr Gln Val Ile Gln Lys Leu
    370                 375                 380
Ala Pro Leu Arg Gln Glu Asn Lys Ala Val Val Tyr Gly Ser Thr Lys
385                 390                 395                 400
Glu Arg Trp Ile Asn Asp Asp Val Leu Ile Tyr Glu Arg Ser Phe Asn
                405                 410                 415
Gly Asp Tyr Leu Leu Val Ala Ile Asn Lys Asn Val Asn Gln Ala Tyr
            420                 425                 430
Thr Ile Ser Gly Leu Leu Thr Glu Met Pro Ala Gln Val Tyr His Asp
        435                 440                 445
Val Leu Asp Ser Leu Leu Asp Gly Gln Ser Leu Ala Val Lys Glu Asn

450                 455                 460
Gly Thr Val Asp Ser Phe Leu Leu Gly Pro Gly Glu Val Ser Val Trp
465                 470                 475                 480
Gln His Ile Ser Glu Ser Gly Ser Ala Pro Val Ile Gly Gln Val Gly
                485                 490                 495
Pro Pro Met Gly Lys Pro Gly Asp Ala Val Lys Ile Ser Gly Ser Gly
            500                 505                 510
Phe Gly Ser Glu Pro Gly Thr Val Tyr Phe Arg Asp Thr Lys Ile Asp
        515                 520                 525
Val Leu Thr Trp Asp Asp Glu Thr Ile Val Ile Thr Leu Pro Glu Thr
    530                 535                 540
Leu Gly Gly Lys Ala Gln Ile Ser Val Thr Asn Ser Asp Gly Val Thr
545                 550                 555                 560
Ser Asn Gly Tyr Asp Phe Gln Leu Leu Thr Gly Lys Gln Glu Ser Val
                565                 570                 575
Arg Phe Val Val Asp Asn Ala His Thr Asn Tyr Gly Glu Asn Val Tyr
            580                 585                 590
Leu Val Gly Asn Val Pro Glu Leu Gly Asn Trp Asn Pro Ala Asp Ala
        595                 600                 605
Ile Gly Pro Met Phe Asn Gln Val Val Tyr Ser Tyr Pro Thr Trp Tyr
    610                 615                 620
Tyr Asp Val Ser Val Pro Ala Asp Thr Ala Leu Glu Phe Lys Phe Ile
625                 630                 635                 640
Ile Val Asp Gly Asn Gly Asn Val Thr Trp Glu Ser Gly Gly Asn His
                645                 650                 655
Asn Tyr Arg Val Thr Ser Gly Ser Thr Asp Thr Val Arg Val Ser Phe
            660                 665                 670
Arg Arg

<210>  2
<211>  2022
<212>  DNA
<213>  Bacillus clarkii 7364
<400>  2
agt aat gca acg aac gat ttg tcg aat gtc aat tat gcg gag gaa gtc      48
Ser Asn Ala Thr Asn Asp Leu Ser Asn Val Asn Tyr Ala Glu Glu Val
1               5                   10                  15

att tat cac att gta aca gat cgg ttt aaa gac gga gat cct gac aac      96
Ile Tyr His Ile Val Thr Asp Arg Phe Lys Asp Gly Asp Pro Asp Asn
            20                  25                  30

aat cct caa gga cag ctg ttt agt aat ggt tgc agt gat ctc aca aag      144
Asn Pro Gln Gly Gln Leu Phe Ser Asn Gly Cys Ser Asp Leu Thr Lys
        35                  40                  45

tat tgc ggt ggt gac tgg cag ggc att atc gat gaa att gaa agc ggt      192
Tyr Cys Gly Gly Asp Trp Gln Gly Ile Ile Asp Glu Ile Glu Ser Gly
    50                  55                  60

tac cta ccg gat atg gga att act gct ctg tgg atc tcc cct cct gtt      240
Tyr Leu Pro Asp Met Gly Ile Thr Ala Leu Trp Ile Ser Pro Pro Val
65                  70                  75                  80

gag aat gta ttt gat tta cat cct gaa ggc ttt tcc tct tat cac ggg      288
Glu Asn Val Phe Asp Leu His Pro Glu Gly Phe Ser Ser Tyr His Gly
                85                  90                  95

tat tgg gcc cga gac ttt aaa aag aca aac cct ttc ttc gga gat ttt      336
Tyr Trp Ala Arg Asp Phe Lys Lys Thr Asn Pro Phe Phe Gly Asp Phe
            100                 105                 110

gat gat ttt tcc cga cta atc gaa aca gct cat gca cat gac ata aaa      384
Asp Asp Phe Ser Arg Leu Ile Glu Thr Ala His Ala His Asp Ile Lys
        115                 120                 125

gta gtt att gat ttt gta cct aac cat act tcc cct gta gac atc gag      432
Val Val Ile Asp Phe Val Pro Asn His Thr Ser Pro Val Asp Ile Glu
    130                 135                 140

gat ggt gca ttg tat gac aac ggt aca tta ctg ggc cac tat tca acg 480
Asp Gly Ala Leu Tyr Asp Asn Gly Thr Leu Leu Gly His Tyr Ser Thr
145                 150                 155                 160

gat gca aac aat tat ttt tat aac tat ggt ggt tca gac ttc tca gac 528
Asp Ala Asn Asn Tyr Phe Tyr Asn Tyr Gly Gly Ser Asp Phe Ser Asp
                165                 170                 175

tat gaa aat agc atc tat cga aac ttg tat gat tta gca agt ctt aac 576
Tyr Glu Asn Ser Ile Tyr Arg Asn Leu Tyr Asp Leu Ala Ser Leu Asn
            180                 185                 190

cag caa cat tcc ttt att gat aaa tac tta aaa gaa tct att caa tta 624
Gln Gln His Ser Phe Ile Asp Lys Tyr Leu Lys Glu Ser Ile Gln Leu
        195                 200                 205

tgg ttg gat acg gga att gac ggg att cgc gtg gat gcg gtt gca cac 672
Trp Leu Asp Thr Gly Ile Asp Gly Ile Arg Val Asp Ala Val Ala His
    210                 215                 220

atg cct ttg ggc tgg caa aaa gca ttt atc tca tct gtc tat gat tac 720
Met Pro Leu Gly Trp Gln Lys Ala Phe Ile Ser Ser Val Tyr Asp Tyr
225                 230                 235                 240

aat cca gtt ttt acc ttt ggt gaa tgg ttt aca gga gca caa ggc agc 768
Asn Pro Val Phe Thr Phe Gly Glu Trp Phe Thr Gly Ala Gln Gly Ser
                245                 250                 255

aat cat tac cac cat ttt gtc aac aac agt ggc atg agc gcc ctt gat 816
Asn His Tyr His His Phe Val Asn Asn Ser Gly Met Ser Ala Leu Asp
            260                 265                 270

ttt cgc tat gct cag gta gcg cag gat gta tta aga aat caa aag gga 864
Phe Arg Tyr Ala Gln Val Ala Gln Asp Val Leu Arg Asn Gln Lys Gly
        275                 280                 285

acg atg cat gac att tac gac atg ttg gca agc act caa tta gat tat 912
Thr Met His Asp Ile Tyr Asp Met Leu Ala Ser Thr Gln Leu Asp Tyr
    290                 295                 300

gag cgg ccg caa gat caa gta acc ttt att gat aat cat gat atc gat 960
Glu Arg Pro Gln Asp Gln Val Thr Phe Ile Asp Asn His Asp Ile Asp
305                 310                 315                 320

cgc ttt acg gtg gaa ggc cga gat aca agg aca acg gac atc gga ctg 1008
Arg Phe Thr Val Glu Gly Arg Asp Thr Arg Thr Thr Asp Ile Gly Leu
                325                 330                 335

gca ttt ctt ttg aca tca aga ggc gta ccg gct att tat tat ggt acg 1056
Ala Phe Leu Leu Thr Ser Arg Gly Val Pro Ala Ile Tyr Tyr Gly Thr
            340                 345                 350

gaa aac tat atg act ggt aaa gga gat cca gga aac aga aaa atg atg 1104
Glu Asn Tyr Met Thr Gly Lys Gly Asp Pro Gly Asn Arg Lys Met Met
        355                 360                 365

gag agc ttt gat caa aca acg aca gcc tat cag gtc atc caa aag ctg 1152
Glu Ser Phe Asp Gln Thr Thr Thr Ala Tyr Gln Val Ile Gln Lys Leu
    370                 375                 380

gca ccg ctc cga caa gaa aat aaa gcg gtg gta tat ggt tca aca aaa 1200
Ala Pro Leu Arg Gln Glu Asn Lys Ala Val Val Tyr Gly Ser Thr Lys
385                 390                 395                 400

gaa cgt tgg att aac gat gat gtg ctc att tat gaa cga tcg ttt aat 1248
Glu Arg Trp Ile Asn Asp Asp Val Leu Ile Tyr Glu Arg Ser Phe Asn
                405                 410                 415

gga gat tat ctt tta gtc gca att aat aaa aat gta aat caa gct tat 1296
Gly Asp Tyr Leu Leu Val Ala Ile Asn Lys Asn Val Asn Gln Ala Tyr
            420                 425                 430

act att tcc ggt ttg ctc acg gaa atg ccc gcg caa gtc tat cat gat 1344
Thr Ile Ser Gly Leu Leu Thr Glu Met Pro Ala Gln Val Tyr His Asp
        435                 440                 445

gtt tta gac agc tta tta gac ggt caa tcg tta gca gta aaa gaa aat 1392
Val Leu Asp Ser Leu Leu Asp Gly Gln Ser Leu Ala Val Lys Glu Asn
    450                 455                 460

ggt aca gtt gat tcc ttt ctg cta gga cca ggt gaa gta agt gta tgg 1440
Gly Thr Val Asp Ser Phe Leu Leu Gly Pro Gly Glu Val Ser Val Trp
465                 470                 475                 480

cag cat ata agt gaa agt ggt tcc gct cct gtt att ggt caa gta ggc 1488
Gln His Ile Ser Glu Ser Gly Ser Ala Pro Val Ile Gly Gln Val Gly
                485                 490                 495

ccg cct atg ggg aaa cct gga gat gct gtg aag att agt ggc agc gga 1536
Pro Pro Met Gly Lys Pro Gly Asp Ala Val Lys Ile Ser Gly Ser Gly
            500                 505                 510

ttt ggt tct gag cct ggc acc gtg tac ttc aga gat acg aaa ata gac 1584
Phe Gly Ser Glu Pro Gly Thr Val Tyr Phe Arg Asp Thr Lys Ile Asp
        515                 520                 525

gtg tta act tgg gat gat gaa acg att gtg atc aca ctg ccg gaa aca 1632
Val Leu Thr Trp Asp Asp Glu Thr Ile Val Ile Thr Leu Pro Glu Thr
    530                 535                 540

tta gga gga aaa gcg caa atc agt gtt act aac tct gac ggc gtg aca 1680
Leu Gly Gly Lys Ala Gln Ile Ser Val Thr Asn Ser Asp Gly Val Thr
545                 550                 555                 560

agt aac ggc tat gat ttt cag ttg ttg aca ggt aag cag gaa tct gtt 1728
Ser Asn Gly Tyr Asp Phe Gln Leu Leu Thr Gly Lys Gln Glu Ser Val
                565                 570                 575

cgt ttc gtt gtg gat aat gcg cat acc aat tat ggg gaa aat gtt tat 1776
Arg Phe Val Val Asp Asn Ala His Thr Asn Tyr Gly Glu Asn Val Tyr
            580                 585                 590

ctt gtt gga aat gtt cct gag ctt ggg aat tgg aac cct gcc gac gca 1824
Leu Val Gly Asn Val Pro Glu Leu Gly Asn Trp Asn Pro Ala Asp Ala
        595                 600                 605

atc gga cca atg ttt aat caa gtc gtt tat tcc tat cca acc tgg tat 1872
Ile Gly Pro Met Phe Asn Gln Val Val Tyr Ser Tyr Pro Thr Trp Tyr
    610                 615                 620

tac gat gtc agt gtt ccc gcg gat acc gcg ttg gaa ttt aag ttt att 1920
Tyr Asp Val Ser Val Pro Ala Asp Thr Ala Leu Glu Phe Lys Phe Ile
625                 630                 635                 640

att gtc gat gga aat gga aat gtt act tgg gaa agc ggg ggt aat cac 1968
Ile Val Asp Gly Asn Gly Asn Val Thr Trp Glu Ser Gly Gly Asn His
                645                 650                 655

aat tat cgt gtt acc tcg gga agc acg gat act gtt cgt gta agt ttt 2016
Asn Tyr Arg Val Thr Ser Gly Ser Thr Asp Thr Val Arg Val Ser Phe
            660                 665                 670

cga agg 2022
Arg Arg

<210> 3
<211> 32

<212>  DNA
<213>  Artificial Sequence
<220>
<223>  Primer
<400>  3
aaagcatgcc attatgagga ggatttaacg tg  32

<210>  4
<211>  33
<212>  DNA
<213>  Artificial Sequence
<220>
<223>  Primer
<400>  4
aaagagctct gcatcggctc tactcatttc atc  33

<210>  5
<211>  34
<212>  DNA
<213>  Artificial Sequence
<220>
<223>  Primer
<400>  5
ggaattactg ctctggcgat ctcccctcct gttg  34

<210>  6
<211>  34
<212>  DNA
<213>  Artificial Sequence
<220>
<223>  Primer
<400>  6
ggaattactg ctctgtgtat ctcccctcct gttg  34

<210>  7
<211>  34
<212>  DNA
<213>  Artificial Sequence
<220>
<223>  Primer
<400>  7
ggaattactg ctctggagat ctcccctcct gttg  34

<210>  8
<211>  34
<212>  DNA
<213>  Artificial Sequence
<220>
<223>  Primer
<400>  8
ggaattactg ctctgtttat ctcccctcct gttg  34

<210>  9
<211>  34
<212>  DNA
<213>  Artificial Sequence
<220>
<223>  Primer
<400>  9
ggaattactg ctctgcacat ctcccctcct gttg  34

<210>  10
<211>  34
<212>  DNA
<213>  Artificial Sequence
<220>
<223>  Primer
<400>  10
ggaattactg ctctgataat ctcccctcct gttg  34

<210>  11
<211>  34
<212>  DNA
<213>  Artificial Sequence
<220>
<223>  Primer
<400>  11
ggaattactg ctctgaagat ctcccctcct gttg  34

<210>  12
<211>  34
<212>  DNA
<213>  Artificial Sequence
<220>
<223>  Primer
<400>  12
ggaattactg ctctgatgat ctcccctcct gttg  34

<210>  13
<211>  34
<212>  DNA
<213>  Artificial Sequence
<220>
<223>  Primer
<400>  13
ggaattactg ctctgcagat ctcccctcct gttg  34

<210>  14
<211>  34
<212>  DNA
<213>  Artificial Sequence
<220>
<223>  Primer
<400>  14
ggaattactg ctctgtcgat ctcccctcct gttg  34

<210>  15
<211>  34
<212>  DNA
<213>  Artificial Sequence
<220>
<223>  Primer
<400>  15
ggaattactg ctctgacgat ctcccctcct gttg  34

<210>  16
<211>  34
<212>  DNA
<213>  Artificial Sequence
<220>
<223>  Primer
<400>  16
ggaattactg ctctggtgat ctcccctcct gttg  34

<210>  17
<211>  34
<212>  DNA
<213>  Artificial Sequence
<220>
<223>  Primer
<400>  17
ggaattactg ctctgtacat ctcccctcct gttg  34

<210>  18
<211>  44
<212>  DNA
<213>  Artificial Sequence
<220>

<223>  Primer
<400>  18
cataaaagta gttattgatt ttccacctaa ccatacttcc cctg  44

<210>  19
<211>  44
<212>  DNA
<213>  Artificial Sequence
<220>
<223>  Primer
<400>  19
cataaaagta gttattgatt tttcacctaa ccatacttcc cctg  44

<210>  20
<211>  44
<212>  DNA
<213>  Artificial Sequence
<220>
<223>  Primer
<400>  20
cataaaagta gttattgatt ttacacctaa ccatacttcc cctg  44

<210>  21
<211>  33
<212>  DNA
<213>  Artificial Sequence
<220>
<223>  Primer
<400>  21
cgcgtggatg cggttcacca catgcctttg ggc  33

<210>  22
<211>  33
<212>  DNA
<213>  Artificial Sequence
<220>
<223>  Primer
<400>  22
cgcgtggatg cggttaaaca catgcctttg ggc  33

<210>  23
<211>  33
<212>  DNA
<213>  Artificial Sequence
<220>
<223>  Primer
<400>  23
cgcgtggatg cggttcgaca catgcctttg ggc  33

<210>  24
<211>  36
<212>  DNA
<213>  Artificial Sequence
<220>
<223>  Primer
<400>  24
catcaagagg cgtaccgtgt atttattatg gtacgg  36

<210>  25
<211>  36
<212>  DNA
<213>  Artificial Sequence
<220>
<223>  Primer
<400>  25
catcaagagg cgtaccggat atttattatg gtacgg  36

<210>  26
<211>  36

<212>  DNA
<213>  Artificial Sequence
<220>
<223>  Primer
<400>  26
catcaagagg cgtaccggag atttattatg gtacgg  36

<210>  27
<211>  36
<212>  DNA
<213>  Artificial Sequence
<220>
<223>  Primer
<400>  27
catcaagagg cgtaccgcat atttattatg gtacgg  36

<210>  28
<211>  36
<212>  DNA
<213>  Artificial Sequence
<220>
<223>  Primer
<400>  28
catcaagagg cgtaccgatt atttattatg gtacgg  36

<210>  29
<211>  36
<212>  DNA
<213>  Artificial Sequence
<220>
<223>  Primer
<400>  29
catcaagagg cgtaccgctt atttattatg gtacgg  36

<210>  30
<211>  36
<212>  DNA
<213>  Artificial Sequence
<220>
<223>  Primer
<400>  30
catcaagagg cgtaccgaat atttattatg gtacgg  36

<210>  31
<211>  36
<212>  DNA
<213>  Artificial Sequence
<220>
<223>  Primer
<400>  31
catcaagagg cgtaccgtct atttattatg gtacgg  36

<210>  32
<211>  36
<212>  DNA
<213>  Artificial Sequence
<220>
<223>  Primer
<400>  32
catcaagagg cgtaccgact atttattatg gtacgg  36

<210>  33
<211>  36
<212>  DNA
<213>  Artificial Sequence
<220>
<223>  Primer
<400>  33
catcaagagg cgtaccgtat atttattatg gtacgg  36

<210>  34
<211>  40
<212>  DNA
<213>  Artificial Sequence
<220>
<223>  Primer
<400>  34
ctatatgact ggtaagggag caccaggaaa cagaaaaatg  40

<210>  35
<211>  40
<212>  DNA
<213>  Artificial Sequence
<220>
<223>  Primer
<400>  35
ctatatgact ggtaagggat gcccaggaaa cagaaaaatg  40

<210>  36
<211>  40
<212>  DNA
<213>  Artificial Sequence
<220>
<223>  Primer
<400>  36
ctatatgact ggtaagggag agccaggaaa cagaaaaatg  40

<210>  37
<211>  40
<212>  DNA
<213>  Artificial Sequence
<220>
<223>  Primer
<400>  37
ctatatgact ggtaagggat ttccaggaaa cagaaaaatg  40

<210>  38
<211>  40
<212>  DNA
<213>  Artificial Sequence
<220>
<223>  Primer
<400>  38
ctatatgact ggtaagggac acccaggaaa cagaaaaatg  40

<210>  39
<211>  40
<212>  DNA
<213>  Artificial Sequence
<220>
<223>  Primer
<400>  39
ctatatgact ggtaagggaa tcccaggaaa cagaaaaatg  40

<210>  40
<211>  40
<212>  DNA
<213>  Artificial Sequence
<220>
<223>  Primer
<400>  40
ctatatgact ggtaagggaa aaccaggaaa cagaaaaatg  40

<210>  41
<211>  40
<212>  DNA
<213>  Artificial Sequence
<220>

<223>  Primer
<400>  41
ctatatgact ggtaagggac tgccaggaaa cagaaaaatg  40

<210>  42
<211>  40
<212>  DNA
<213>  Artificial Sequence
<220>
<223>  Primer
<400>  42
ctatatgact ggtaagggaa tgccaggaaa cagaaaaatg  40

<210>  43
<211>  40
<212>  DNA
<213>  Artificial Sequence
<220>
<223>  Primer
<400>  43
ctatatgact ggtaagggaa acccaggaaa cagaaaaatg  40

<210>  44
<211>  40
<212>  DNA
<213>  Artificial Sequence
<220>
<223>  Primer
<400>  44
ctatatgact ggtaagggac ccccaggaaa cagaaaaatg  40

<210>  45
<211>  40
<212>  DNA
<213>  Artificial Sequence
<220>
<223>  Primer
<400>  45
ctatatgact ggtaagggac aaccaggaaa cagaaaaatg  40

<210>  46
<211>  40
<212>  DNA
<213>  Artificial Sequence
<220>
<223>  Primer
<400>  46
ctatatgact ggtaagggaa gcccaggaaa cagaaaaatg  40

<210>  47
<211>  40
<212>  DNA
<213>  Artificial Sequence
<220>
<223>  Primer
<400>  47
ctatatgact ggtaagggaa ccccaggaaa cagaaaaatg  40

<210>  48
<211>  40
<212>  DNA
<213>  Artificial Sequence
<220>
<223>  Primer
<400>  48
ctatatgact ggtaagggag tgccaggaaa cagaaaaatg  40

<210>  49
<211>  40

<212>  DNA
<213>  Artificial Sequence
<220>
<223>  Primer
<400>  49
ctatatgact ggtaagggat ggccaggaaa cagaaaaatg  40

<210>  50
<211>  40
<212>  DNA
<213>  Artificial Sequence
<220>
<223>  Primer
<400>  50
ctatatgact ggtaagggat atccaggaaa cagaaaaatg  40

## Description

### [Cross-reference to related patent applications]

The present application claims priority under Japanese Patent Application 2006-160175, filed on June 8, 2006, the entire contents of which are hereby incorporated by reference.

### [Technical Field]

The present invention relates to a mutant cyclodextrin glucanotransferase having improved, highly specific activity, a gene encoding this mutant, and a method for manufacturing the mutant employing the gene.

### [Background Art]

Cyclodextrin glucanotransferase (CGTase; EC 2.4.1.19) is an enzyme that acts on starches such as *alpha*-1,4-glucans, producing cyclodextrins (CD), cyclic maltooligosaccharides bound by *alpha*-1 ,4-glucopyranoside bonds, by means of an intramolecular transfer reaction. The CDs that are produced from starches and the like by CGTase are primarily *alpha*-CD, *beta*-CD, and *gamma*-CD comprised of 6 to 8 glucose units. These CDs have the ability to form clathrates with numerous molecules (guest compounds), and are extremely useful in the areas of foods, pharmaceuticals, agricultural chemicals, cosmetics, daily sundries, and chemical products because they change the various chemical, physical, and physiological properties of the guest compounds.

Based on the main components of the CD produced from starch and the like, CGTase can be divided into *alpha*-CGTase, which preferentially produces *alpha*-CD; *beta*-CGTase, which preferentially produces *beta*-CD; *alphalbeta-*CGTase, which preferentially produces *alpha*-CD and *beta*-CD; and *gamma*-CD, which preferentially produces *gamma*-CGTase. Many of the CGTases that have been reported thus far have been *alpha*-CGTases, *beta*-CGTases, and *alphalbeta*-CGTases. There have been few reports of *gamma*-CGTases. Of the enzymes that have been reported to be *gamma*-CGTases, some exhibit accelerated *beta*-CD production rates during the latter stages of the reaction, while others produce *beta*-CD in an amount that is equal or greater than the amount of *gamma*-CD produced. Since the quantity of *gamma*-CD produced decreases markedly in a highly concentrated substrate of 10 percent or more, it is necessary to add various organic solvents such as ethanol to the *gamma*-CD producing reaction to increase the quantity of *gamma*-CD produced. Since this method requires a tedious step of removing the solvent or the like, it does not lend itself to the safe, inexpensive industrial production of *gamma*-CD and compositions containing *gamma*-CD.

To solve these problems, attempts have been made to change the structural gene of CGTase and improve the quantity of *gamma*-CD produced (for example, see Akira Nakamura, Keiko Haga, and Kunio Yamane, Biochemistry, 32, 6624-6631, 1993; Michio Kubota, Yoshiki Matsuura, Shuzo Sakai, and Yukiteru Kutsume, Oyo Toshitsu Kagaku, 41 (2), 245-253, 1994; Japanese Translation of PCT International Application (TOKUHYO) No. 2003-531564; Japanese Translation of PCT International Application (TOKUHYO) Heisei No. 11-503906; Japanese Unexamined Patent Publication (KOKAI) Heisei No. 10-33187; the entire contents of which are hereby incorporated by reference). However, these attempts have resulted in decreased specific activity in CD synthesis and marked reduction in the quantity of *beta*-CD produced by the original activity despite an increase in the quantity of *gamma*-CD produced, and are inadequate from an industrial perspective. As a result, *alpha*-CD and *beta-*CD are employed in a variety of fields, but gamma-CD is currently seldom employed.

The same holds true for compositions containing CD. Compositions containing primarily alpha or *beta*-CD are employed in a variety of fields. However, there are few examples of the use of compositions containing primarily *gamma*-CD. Since the CD composition of a CD-containing composition is determined exclusively by whether the CGTase used to prepare it is *alpha, beta*, or *gamma*-CGTase, it is difficult to prepare a CD-containing composition of desired CD composition.

Prompted by these circumstances, *Bacillus clarkii* strain 7364 was discovered to produce a new *gamma*-CGTase the main product of which is *gamma*-CD, the gene encoding this enzyme was sequenced, the amino acid sequence of the enzyme was determined, a method for manufacturing *gamma-*CD employing this enzyme was established, a method for manufacturing a CD-containing composition of desired CD composition was established, and patent applications were filed (for example, see Japanese Unexamined Patent Publication (KOKAI) Nos. 2003-102489, 2001-327299, and 2001-327284, the entire contents of which are hereby incorporated by reference).

However, the specific activity of the *gamma*-CGTase that has been reported thus far has been relatively low. Thus, when producing *gamma*-CD and CD-containing compositions of desired CD composition on an industrial scale, a large quantity of *gamma*-CGTase is required, necessitating large-scale microbial culturing to produce the enzyme. There is also a major problem of economy in the form of an increased load generated in the purification step by the saccharified solution following the CD production reaction, resulting from the large quantity of enzyme required to obtain a targeted composition.

Accordingly, one object of the present invention is to provide an improved mutant CGTase that is imparted with greater specific activity than the parent *gamma*-CGTase by the addition of a mutation to *gamma*-CGTase, reducing the quantity of enzyme required to obtain a targeted CD composition.

A further object of the present invention is to provide a gene encoding this mutant CGTase and a method for manufacturing the mutant CGTase employing this gene.

### Disclosure of the Invention

To solve the above-stated problems, the present inventors examined mutant CGTases having better specific activity than the parent *gamma*-CGTase. As a result, they discovered that a mutant CGTase in which amino acid residues at specific positions in the amino acid sequence of *gamma*-CGTase had been replaced with another amino acid residues had greater specific activity than the parent *gamma*-CGTase. The present invention was devised on that basis.

The present invention relates to an improved mutant cyclodextrin glucanotransferase, having greater specific activity in the synthesis of *gamma-*cyclodextrin than the parent *gamma-*cyclodextrin glucanotransferase, in which at least one of the amino acid residues in the parent *gamma-*cyclodextrin glucanotransferase has been replaced with an amino acid residue differing from that particular amino acid residue in the parent *gamma*-cyclodextrin glucanotransferase.

The following are desirable forms of the mutant of the present invention:
1) a mutant in which, in the above parent *gamma*-cyclodextrin glucanotransferase having the amino acid sequence denoted by SEQ ID NO: 1 in the Sequence Listing, at least one of the amino acid residues at positions 75, 82, 91, 92, 119, 134, 147, 151, 223, 225, 234, 320, 347, 359, 360, 361, 451, 625, 656, and 662 in the amino acid sequence denoted by SEQ ID NO: 1 in the Sequence Listing has been replaced with an amino acid residue differing from the amino acid residue indicated for the corresponding position in the amino acid sequence of SEQ ID NO: 1;
2) the above mutant, wherein the differing amino acid residue(s) is/are:
   Ala, Cys, Gln, Glu, His, Ile, Leu, Lys, Met, Phe, Ser, Thr, Tyr, or Val at position 75;
   Asp at position 82;
   Leu at position 91;
   Thr at position 92;
   Val at position 119;
   Ala, Pro, Ser, or Thr at position 134;
   Thr at position 147;
   Asp at position 151;
   Arg, His, Lys, or Val at position 223;
   Leu at position 225;
   Asn at position 234;
   Glu at position 320;
   Asn, Asp, Cys, Glu, His, Ile, Leu, Ser, Thr, Tyr, or Val at position 347;
   Gln at position 359;
   Arg at position 360;
   Ala, Asn, Cys, Gln, Gly, Glu , His, Ile, Leu, Lys, Met, Phe, Pro, Ser, Thr, Trp, Tyr, or Val at position 361;
   Val at position 451;
   Cys at position 625;
   Tyr at position 656; and
   Leu at position 662;
3) a mutant in which, in the amino acid sequence denoted by SEQ ID NO: 1 in the Sequence Listing, one or a few deletions, substitutions, or additions have been made to amino acid residues other than those at positions 75, 82, 91, 92, 119, 134, 147, 151, 223, 225, 234, 320, 347, 359, 360, 361, 451, 625, 656, and 662;
4) the above mutant, in which in the parent *gamma*-cyclodextrin glucanotransferase having an amino acid sequence having 60 percent or greater homology with the amino acid sequence denoted by SEQ ID NO: 1 of the Sequence Listing, at least one amino acid residue among the amino acid residues corresponding to positions 75, 82, 91, 92, 119, 134, 147, 151, 223, 225, 234, 320, 347, 359, 360, 361, 451, 625, 656, and 662 in the amino acid sequence of the parent *gamma*-cyclodextrin glucanotransferase denoted by SEQ ID NO: 1 in the Sequence Listing has been replaced with an amino acid residue differing from the amino acid residue at the corresponding position in the amino acid sequence of the parent *gamma*-cyclodextrin glucanotransferase;
5) the above mutant, the specific activity of which as measured at a pH between pH 6 to 11, is 1.1-fold or greater the specific activity of the parent *gamma-*cyclodextrin glucanotransferase; and
6) the above mutant, the specific activity of which as measured at pH 7.5 or pH 10, is 1.1-fold or greater the specific activity of the parent *gamma*-cyclodextrin glucanotransferase.

The present invention further relates to a gene encoding the amino acid sequence of the above-described mutant of the present invention, to a recombinant vector comprising the gene of the present invention, and to a transformant that has been transformed or modified by chromosomal homologous recombination with the recombinant vector of the present invention.

Still further, the present invention relates to a method for manufacturing the mutant of the present invention comprising: culturing the transformant of the present invention, and collecting the mutant cyclodextrin glucanotransferase produced by the transformant.

The present invention provides an improved mutant CGTase having higher specific activity than the parent *gamma*-CGTase. Using this mutant, it is possible to increase the quantity of CD produced or decrease the quantity of enzyme employed in the course of manufacturing a CD-containing composition. The mutant is extremely useful industrially as a ***gamma***-CD producing enzyme.

### Best Modes of Carrying Out the Invention

The present invention is an improved mutant CGTase, in which at least one of the amino acid residues of the parent *gamma*-CGTase has been substituted with an amino acid residue differing from the corresponding amino acid residue in the parent *gamma*-CGTase, having higher specific activity in the synthesis of *gamma*-CD than the parent *gamma*-CGTase.

In the present invention, the term "parent *gamma*-CGTase" means CGTase having activity permitting the production of *gamma*-CD of about 50 to 100 weight percent of the CD produced when acting on starch or a starch-derived substrate. Examples of such parent *gamma*-CGTase are *gamma*-CGTase having the amino acid sequence denoted by SEQ ID NO: 1 of the Sequence Listing, and *gamma*-CGTase differing from this *gamma*-CGTase . It may be wild type, a wild-type mutant, or a mutant obtained by artificial mutation.

The term "starch or a starch-derived substrate" means the following. Examples of starches are those obtained from potatoes, corn, sweet potatoes, wheat, rice, tapioca, palm, and the like. These can be glutinous, nonglutinous, or high-amylose varieties. Examples of starch-derived substrates are amylose, amylopectin, soluble starch, dextrin, starch syrup, and maltooligosaccharides; so long as *gamma*-CD is produced when subjected to the action of *gamma-*CGTase, any of these can be used.

In one aspect of the mutant of the present invention, the parent *gamma-*CGTase is a mutant CGTase having the amino acid sequence denoted by SEQ ID NO: 1 in the Sequence Listing. In this mutant, at least one of the amino acid residues at positions 75, 82, 91, 92, 119, 134, 147, 151, 223, 225, 234, 320, 347, 359, 360, 361, 451, 625, 656, and 662 in the amino acid sequence denoted by SEQ ID NO: 1 in the Sequence Listing has been replaced with an amino acid residue differing from the amino acid residue indicated for the corresponding position in the amino acid sequence of SEQ ID NO: 1. Further, the mutant is an improved mutant CGTase having greater specific activity than the parent *gamma-*CGTase having the amino acid sequence of SEQ ID NO: 1 in the Sequence Listing.

An example of a *gamma*-CGTase having the amino acid sequence denoted by SEQ ID NO: 1 in the Sequence Listing is the *gamma*-CGTase derived from *Bacillus clarkii* strain 7364 (FERM BP-7156).

### Notation

In the present Specification and in the Claims, the conventional single-letter and three-letter codes are employed for amino acid residues. To facilitate citation, the mutant CGTase of the present invention will be described using the following notation:
Original amino acid: position: substituted amino acid Based on this notation, substitution of the tryptophan at position 75 with leucine would be indicated by: Trp75Leu, or W75L.

Multiple mutations are separated by "/" marks. For example, the replacement of tryptophan at position 75 with leucine and the asparagine at position 82 with aspartic acid would be indicated by: Trp75Leu/Asn82Asp, or W75UN82D.

The amino acid residues at the above positions in the parent *gamma-*CGTase comprised of the amino acid sequence denoted by SEQ ID NO: 1 are: position 75: Trp; position 82: Asn; position 91: Phe; position 92: Ser; position 119: Ile; position 134: Val; position 147: Ala; position 151: Asn; position 223: Ala; position 225: Met; position 234: Ile, position 320: Asp; position 347: Ala; position 359: Lys; position 360: Gly; position 361: Asp; position 451: Asp; position 625: Tyr; position 656: His; and position 662: Ser.

In the mutant CGTase of the present invention, the amino acid residues at the above positions are substituted with amino acid residues differing from the amino acid residues indicated at the corresponding positions in the amino acid sequence denoted by SEQ ID NO: 1. There are a total of 20 amino acid residues: Ala, Asn, Asp, Arg, Cys, Gln, Glu, Gly, His, Ile, Leu, Lys, Met, Phe, Pro, Ser, Thr, Trp, Tyr, and Val. For example, the amino acid residue present at position 75 in the amino acid sequence denoted by SEQ ID NO: 1 in the wild type enzyme is Trp; in the mutant, it can be selected from among the 19 amino acids other than Trp. The same applies to the amino acid residues at positions other than position 75. However, an amino acid residue resulting in a higher specific activity than that of the parent *gamma*-CGTase is substituted into the mutant.

Amino acid residues that are desirably substituted for various amino acid residues (amino acid residue sequences in which the amino acid residues differ at the corresponding positions) are indicated below. As is specifically indicated in the embodiments, mutants having the amino acid substitutions indicated below have specific activity that is at least 10 percent (1.1-fold) higher than the specific activity of the parent *gamma*-CGTase.

Ala, Cys, Gln, Glu, His, Ile, Leu, Lys, Met, Phe, Ser, Thr, Tyr, or Val is desirable at position 75.
Asp is desirable at position 82.
Leu is desirable at position 91.
Thr is desirable at position 92.
Val is desirable at position 119.
Ala, Pro, Ser, or Thr is desirable at position 134.
Thr is desirable at position 147.
Asp is desirable at position 151.
Arg, His, Lys, or Val is desirable at position 223.
Leu is desirable at position 225.
Asn is desirable at position 234.
Glu is desirable at position 320.
Asn, Asp, Cys, Glu, His, Ile, Leu, Ser, Thr, Tyr, or Val is desirable at position 347.
Gln is desirable at position 359.
Arg is desirable at position 360.
Ala, Asn, Cys, Gln, Gly, Glu, His, Ile, Leu, Lys, Met, Phe, Pro, Ser, Thr, Trp, Tyr, or Val is desirable at position 361.
Val is desirable at position 451.
Cys is desirable at position 625.
Tyr is desirable at position 656.
Leu is desirable at position 662.

The mutant CGTase of the present invention not only includes the substitution of other amino acids for any of the amino acid residues at any of the positions corresponding to the particular positions indicated above, but so long as the characteristic improvement in the form of heightened specific activity is present, also includes the deletion, substitution or addition of one or a few amino acid residues at other positions in this amino acid sequence. In such cases, the basis for comparison of specific activity is the parent *gamma*-CGTase without the deletion, substitution or addition.

In a further aspect of the mutant of the present invention, the mutant is one in which the parent *gamma*-CGTase is a *gamma*-CGTase that differs from the *gamma*-CGTase having the amino acid sequence denoted by SEQ ID 1 in the Sequence Listing. An example of the *gamma*-CGTase that differs from the *gamma*-CGTase having the amino acid sequence denoted by SEQ ID 1 in the Sequence Listing is wild type, or a wild-type mutant, having activity resulting in about 50 to 100 weight percent of the CD produced being *gamma*-CD when acting on a starch or starch-derived substrate, and having an amino acid sequence with about 60 percent or greater homology with the amino acid sequence denoted by SEQ ID NO: 1 of the Sequence Listing. In particular, an enzyme is desirable that is comprised of an amino acid sequence having 60 percent or greater homology with the amino acid sequence denoted by SEQ ID NO: 1 of the Sequence Listing; that has one or a few amino acid residue deletions or substitutions relative to *alpha* or *beta*-CGTase, such as *alpha-*CGTase (Genebank Accession No. AAA22298)) derived from *Bacillus macerans* or beta-CGTase (Genebank Accession No. CAA55023) derived from *Bacillus circulans* strain 251 at subsite -3 and/or -7 of subsites +2 to -7 of the substrate binding sites identified by beta-CGTase and the like derived from *Bacillus circulans* strain 251; and that has activity resulting in about 50 to 100 weight percent of the CD produced being *gamma*-*CD* when acting on a starch or starch-derived substrate.

This mutant is an improved mutant CGTase having greater specific activity than the parent *gamma*-CGTase and having an amino acid sequence in which at least one of the amino acid residues corresponding to positions 75, 82, 91, 92, 119, 134, 147, 151, 223, 225, 234, 320, 347, 359, 360, 361, 451, 625, 656, and 662 in the amino acid sequence of the amino acid sequence of the parent *gamma*-CGTase denoted by SEQ ID NO: 1 of the Sequence Listing has been replaced by an amino acid residue differing from the amino acid residues indicated for the corresponding position in the amino acid sequence of the parent *gamma*-CGTase.

The amino acid residues of corresponding positions can be specified and the homology of amino acid sequences can be calculated by comparing amino acid sequences with a known algorithm such as the Lippman-Parson algorithm and giving maximum homology to the retained amino acid residues that are present in the amino acid sequences of the various CGTases. By aligning the amino acid sequences of CGTase in this manner, it is possible to determine the positions of homologous amino acid residues within the sequences of various CGTases. Homologous positions are thought to be present at identical positions within three-dimensional structures, and can be presumed to have similar effects in terms of the specific functions of the targeted CGTase.

Mutants are desirable in which the amino acid residues at positions corresponding to positions 75, 82, 91, 92, 119, 134, 147, 151, 223, 225, 234, 347, 359, 360, 361, 451, 625, 656, and 662 in a parent *gamma*-CGTase comprised of an amino acid sequence having 60 percent or greater homology with the amino acid sequence denoted by SEQ ID NO: 1 of the Sequence Listing are Trp, Asn, Phe, Ala, Ile, Val, Ala, Asn, Ala, Met, Val, Thr, Asp, Gly, Asp, Ser, Tyr, His, and Glu, respectively. A mutant is desirable in which the amino acid residue at the position corresponding to position 320 is additionally Asn or Asp.

The amino acid residues corresponding to positions 75 to 662 of the amino acid sequence denoted by SEQ ID NO: 1 in the Sequence Listing of *gamma*-CGTase derived from *Bacillus* sp. 290-3 and *gamma*-CGTase derived from *Bacillus* sp. G-825-6 are given in Table 1. The amino acid sequence of CGTase derived from *Bacillus* sp. 290-3 is based on Genebank Accession No. CAA01436 and the amino acid sequence of CGTase derived from Bacillus sp. G-825-6 is based on Genebank Accession No. AB201304.

[Table 1]

**Table 1 Amino acid residues corresponding to positions 75 to 662**

| Position | Derived from *Bacillus clarkii* strain 7364 | Derived from *Bacillus sp.* 290-3 | Derived from *Bacillus sp.* G-825-6 |
|---|---|---|---|
| 75 | 75Trp | 72Trp | 72Trp |
| 82 | 82Asn | 79Asn | 79Asn |
| 91 | 91Phe | 88Phe | 88Phe |
| 92 | 92Ser | 89Ala | 89Ala |
| 119 | 119Ile | 116Ile | 116Ile |
| 134 | 134Val | 131Val | 131Val |
| 147 | 147Ala | 144Ala | 144Ala |
| 151 | 151Asn | 148Asn | 148Asn |
| 223 | 223Ala | 220Ala | 220Ala |
| 225 | 225Met | 222Met | 222Met |
| 234 | 234Ile | 231Val | 231Val |
| 320 | 320Asp | 317Asp | 317Asn |
| 347 | 347Ala | 344Thr | 344Thr |
| 359 | 359Lys | 356Asp | 356Asp |
| 360 | 360Gly | 357Gly | 357Gly |
| 361 | 361Asp | 358Asp | 358Asp |
| 451 | 451Asp | 448Ser | 448Ser |
| 625 | 625Tyr | 622Tyr | 622Tyr |
| 656 | 656His | 653His | 653His |
| 662 | 662Ser | 659Glu | 659Glu |

The mutant CGTase of the present invention is a mutant having an amino acid sequence with 60 percent or greater homology with the amino acid sequence of the *gamma*-CGTase denoted by SEQ ID NO: 1 of the Sequence Listing, in which the amino acid residues at the above-listed positions are replaced with amino acid residues differing from the amino acid residues indicated for the corresponding positions. There are a total of 20 amino acid residues: Ala, Asn, Asp, Arg, Cys, Gln, Glu, Gly, His, Ile, Leu, Lys, Met, Phe, Pro, Ser, Thr, Trp, Tyr, and Val. For example, the amino acid residue present at position 75 in the amino acid sequence denoted by SEQ ID NO: 1 in the wild type enzyme is Trp; in the mutant, it can be selected from among the 19 amino acids other than Trp. The same applies to the amino acid residues at positions other than position 75. However, an amino acid residue resulting in a higher specific activity than that of the parent *gamma*-CGTase is substituted into the mutant.

In the mutant of the present invention, it is possible for amino acid residues to be substituted simultaneously at two or more positions so long as the characteristic improvement of higher specific activity than the parent *gamma-*CGTase is present.

In the present invention, the "specific activity" is the level of *gamma*-CD producing activity of a prescribed quantity of protein. The quantity of protein can be measured by the method described in Embodiment 2-3. The specific activity is the value calculated for CGTase activity in the production of *gamma*-CD as measured under conditions of a pH between pH 6 and 11, desirably pH 7.5 or pH 10, for example, at a temperature of 20 to 65°C, preferably 30 to 60°C, and more preferably, 40 to 55°C, for 5 to 30 minutes, desirably 10 to 15 minutes. The CGTase activity in the production of *gamma*-CD changes based on the pH of the reaction solution, the temperature, and the like. Typical methods of measuring and calculating the specific activity in the production of *gamma*-CD are specifically described in the embodiments. More specifically, CGTase activity can be measured for calculating the specific activity by the method described in Embodiment 2-4. That is, measurement is conducted under conditions of pH 7.5 or 10, a temperature of 40°C, for 10 minutes.

In the present invention, the statement that the specific activity is greater than that of the parent *gamma*-CGTase means that the specific activity of CGTase in the production of *gamma*-CD is, for example, higher by 10 percent (1.1-fold) or more, preferably higher by 30 percent (1.3-fold) or more, more preferably higher by 50 percent (1.5-fold) or more, and optimally, higher by 100 percent (double) or more, relative to the specific activity of the parent *gamma-*CGTase.

The mutant CGTase of the present invention can be obtained by the following method, for example. That is, a gene (for example, having the nucleotide sequence denoted by SEQ ID NO: 2) encoding a cloned parent *gamma*-CGTase (for example, CGTase having the amino acid sequence denoted by SEQ ID NO: 1) is subjected to substitution (also referred to as "mutation" hereinafter), the mutant gene obtained is employed to transform a suitable host, the transformed host is cultured, and mutant CGTase is collected from the culture.

The gene encoding the parent *gamma*-CGTase can be obtained from the chromosome of *Bacillus clarkii* strain 7364 (FERM BP-7156) for example, by the shotgun method or PCR, and can be cloned by any of the usual gene recombination techniques.

Methods such as commonly conducted random mutation and site-specific mutation can be employed as the means of mutating the gene encoding the parent *gamma*-CGTase. More specifically, error prone PCR, recombinant PCR, or the like can be employed for mutation.

The mutant CGTase of the present invention employing the mutant gene that has been obtained can be produced by incorporating the gene into a vector that is capable of being copied and maintained in a host bacterium, capable of being made to stably express the enzyme, and capable of stably retaining the gene, and using the recombinant vector that has been obtained to transform a host bacterium.

Examples of such a vector when employing *E. coli* as host are pUC19 and pHY300PLK. Examples when employing *Bacillus subtilis* as host are pUB110, pHY300PLK, and PAMa/*pha*1.

The competent cell method, electroporation method, protoplast method, or the like can be employed to transform the host bacterium. Examples of host bacteria are Gram-negative bacteria such as *E*. *coli,* Gram-positive bacteria of the genus *Bacillus* (*Bacillus subtilis*), actinomycetes of the genus *Streptomyces*, yeasts of the genus *Saccharomyces*, and molds of the genus *Aspergillus*.

The transformant obtained can be cultured under suitable conditions using a medium containing a utilizable carbon source, nitrogen source, metal salts, vitamins, and the like. The culturing temperature and duration can be suitably determined taking into account the optimal conditions of the transformant employed. Following culturing, the usual methods of fractionation and purification can be employed to obtain purified enzyme from the culture solution.

The mutant CGTase of the present invention thus obtained has greater specific activity than the parent *gamma*-CGTase. A method of preparing the mutant CGTase of the present invention by genetic engineering techniques has been set forth above. In addition to employing the genetic engineering technique of inducing mutation in parent *gamma*-CGTase, the mutant CGTase of the present invention can also be obtained by collecting natural CGTase containing such mutations from the natural world. Such natural CGTase is also covered by the mutant CGTase of the present invention.

The mutant CGTase of the present invention that is obtained has greater specific activity than the parent *gamma*-CGTase, and in the course of manufacturing a composition containing *gamma*-CD, is useful as a *gamma*-CD producing enzyme that increases the quantity of *gamma*-CD produced or decreases the quantity of *gamma*-CGTase employed.

There are cases in which the mutant CGTase of the present invention enhances specific activity in the production of *alpha*-CD and/or *beta*-CD in addition to enhancing specific activity in the production of *gamma*-CD through the introduction of mutations in the amino acid residues. The mutant CGTase of the present invention also includes such mutant CGTase so long as there is an increase of 10 percent (1.1-fold) or more in specific activity in the production of *gamma*-CD relative to the specific activity of the parent *gamma*-CGTase.

When causing CGTase to act on starch or a starch-derived substrate, the ratio of the various quantities of *alpha*-CD, *beta*-CD, and *gamma*-CD that are produced varies with reaction conditions such as the reaction duration. With the mutant CGTase of the present invention, a change in the ratio of the quantity of CD produced will sometimes occur due to the introduction of amino acid residue mutations. The quantity or ratio of *gamma*-CD produced is sometimes lowered relative to the quantity or ratio of *alpha*-CD and/or *beta*-CD produced due to the reaction conditions that are set. However, even in such cases, the quantity or ratio of *gamma*-CD produced by the mutant CGTase of the present invention can be increased by optimizing the reaction conditions.

### Embodiments

Embodiments of the present invention are described in detail below. However, it will be understood that the embodiments set forth below are examples and are not intended to limit the present invention.

### Embodiment 1

### Preparation of mutant CGTase

Error prone PCR was conducted on an about 2.2 kb range running from 20 base pairs upstream from the start codon of a CGTase structural gene derived from *Bacillus clarkii* strain 7364 to a stop codon 100 base pairs downstream using a primer 1 (SEQ ID NO: 3), primer 2 (SEQ ID NO: 4) and Takara *Taq* (Takara) capable of amplifying this about 2.2 kb fragment of DNA to impart random mutation. An *Sph*I linker was provided on the 5' terminal side of the sense strand of primer 1 and an *Sa*cI linker was provided on the 5' terminal side of the antisense strand of primer 2. The PCR conditions were as follows: after denaturing the template DNA for 5 min at 94°C, 30 reaction cycles were conducted, each of which consisted of 1 min at 94°C, 1.5 min at 55°C, and 3 min at 74°C. The amplified DNA fragments were purified using a GFX PCR DNA and Gel Band Purification Kit (Amersham Biosciences) and the terminal restrictase linkers were cleaved with *Sph*I and *Sac*I. Following cleavage, the DNA fragments were purified using a GFX PCR DNA and Gel Band Purification Kit. The purified DNA fragments were combined with pUC19 that had been processed with *Sph*I and *Sac*I, after which a ligase reaction was conducted with Ligation High (Toyobo). After the ligase reaction, the solution was used to transform a host bacterium in the form of *E. coli* JM109.

The E. coli JM109 transformant was cultured for 14 to 20 hours at 30°C on LB-AG agar medium (polypeptone (Difco) 1 percent (w/v), yeast extract (Difco) 0.5 percent (w/v), sodium chloride 1 percent (w/v), ampicillin 50 ppm, glycose 0.5 percent (w/v), agar 1.5 percent (w/v)). Isopropyl-*beta*-D-thiogalactopyranoside (IPTG)-containing starch azure agar (starch azure (Sigma) 0.5 (w/v), soluble starch 0.5 percent (w/v), agar 1.5 percent (w/v), 0.1 mM IPTG, 25 mM HEPES-NaOH (pH 7.5)) was layered on the LB-AG agar medium on which the *E*. *coli* JM109 transformant had been cultured and a reaction was conducted for 4 to 8 hours at 30°C. Candidate strains of transformants producing improved mutant CGTase with greater specific activity than the parent *gamma*-CGTase were obtained based on the formation of halos.

### Embodiment 2

### Evaluation of mutant CGTase

### Embodiment 2-1

### Nucleotide sequencing of gene encoding mutant CGTase

The candidate strains were inoculated onto 2 mL of LB-A medium (polypeptone (Difco) 1 percent (w/v), yeast extract (Difco) 0.5 percent (w/v), sodium chloride 1 percent (w/v), ampicillin 50 ppm) and cultured for 12 to 16 hours at 30°C. Plasmid was then recovered from the bacterial mass obtained by centrifugal separation using a GFX *Micro* Plasmid Prep Kit (Amersham Biosciences). The nucleotide sequence of the gene encoding the mutant CGTase inserted in the plasmid that was recovered was sequenced with a MegaBACE 1000 Multicapillary System (Amersham Biosciences).

### Embodiment 2-2 Purification of mutant CGTase

The above candidates were transplanted onto 5 mL of LB-A medium, cultured for 12 to 20 hours at 30°C, transplanted onto 300 mL of LB-A medium, and shake cultured at 30°C. When the turbidity at 600 nm reached 0.4 to 1.0, IPTG was added to a final concentration of 0.1 mM and culturing was conducted for another 12 to 20 hours. The bacterial mass obtained by centrifugal separation from the culture solution was subjected to 5 minutes of ultrasonic disruption in an Ultrasonic Disruptor UD-201 (Tomy), and a crude enzyme solution of the bacterial mass was obtained by centrifugal separation from the sonicated solution. This crude enzyme solution was electrophoretically purified to a homogenous level by affinity chromatography using immobilized *gamma*-CD Sepharose6B consisting of *gamma*-CD immobilized on Sepharose6B (Amersham Biosciences).

### Embodiment 2-3 Measurement of protein concentration

The protein concentration of the purified CGTase was measured with a DC protein assay kit (BioRad). Bovine serum albumin was employed as the standard protein.

### Embodiment 2-4 Measurement of CGTase activity

Four hundred and fifty microliter quantities of 1.5 percent soluble starch solution/various buffer solutions (pH 5-7: 1/4 x McIIvaine buffer solution, pH 7.5-8.5: 25 mM HEPES-NaOH buffer solution, pH 9-10.5: 25 mM Gly-NaCl-NaOH buffer solution, pH 11-11.9: 25 mM Na₂HPO₄-NaOH buffer solution) were maintained at 40°C and 50 microliter quantities of suitably diluted enzyme solution were added to start the reaction. Ten minutes after the reaction began, 500 microliter quantities of 0.05 N HCl were added to stop the reaction. To the reaction was admixed 5 mM BCG solution (in 20 percent ethanol) , the mixture was maintained at room temperature for 20 minutes, 2 mL of 1 M BCG buffer solution (pH 4.2) was added, and the absorbance was measured at 630 nm. The *gamma*-CD content in the reaction solution was determined from the absorbance value based on a calibration curve prepared in advance. One unit of CGTase activity was defined as the quantity of enzyme producing 1 micromol of gamma-CD per unit time under the above reaction conditions.

### Embodiment 2-5

### Method of evaluating mutant CGTase

The specific activity was calculated from the protein concentration and the CGTase activity value of the purified mutant CGTase derived from the above candidate strains. This specific activity was then compared to the specific activity of CGTase derived from *Bacillus clarkii* strain 7364 (parent CGTase) to select mutant CGTase having improved specific activity. Mutation sites specified based on determination of the nucleotide sequence of the gene encoding the mutant CGTase selected were adopted as mutation sites that afforded improvement.

### Embodiment 2-6

### Evaluation of mutant CGTase

The CGTase activity and protein concentration of the parent CGTase and various mutant CGTases were measured at pH 7.5 and 10.0 and the relative activities were calculated. Table 2 gives the measured values of specific activity and the relative value of the mutant CGTase of the present invention when the specific activity of the parent CGTase was denoted as 100. The specific activity of the parent CGTase was 0.47 U/mg at pH 7.5 and 5.41 U/mg at pH 10.0. By contrast, the specific activity of the various mutant CGTases was 0.39 to 4.35 U/mg at pH 7.5 and 2.89 to 9.28 U/mg at pH 10.0. Mutant CGTases were obtained with specific activity enhanced by 1.1-fold or more at pH 7.5 or pH 10.0, or both.

[Table 2]

**Table 2**

| CGTase specific activity at pH 7.5 and 10.0 of parent CGTase and various mutant CGTases | | | | | |
|---|---|---|---|---|---|
| | | pH 7.5 | | pH 10.0 | |
| | | Specific activity (U/mg) | Relative value (%) | Specific activity (U/mg) | Relative value (%) |
| Comp. Ex. | Parent CGTase | 0.47 | 100 | 5.41 | 100 |
| Invention | W75L | 3.49 | 743 | 8.41 | 155 |
| | N82D | 0.59 | 126 | 5.94 | 110 |
| | F91 L | 0.71 | 151 | 9.28 | 172 |
| | S92T | 0.62 | 132 | 5.05 | 93 |
| | I119V | 0.58 | 123 | 5.87 | 109 |
| | V134A | 1.00 | 213 | 5.15 | 95 |
| | A147T | 0.58 | 123 | 5.40 | 100 |
| | N151D | 0.43 | 91 | 6.46 | 119 |
| | A223V | 0.51 | 109 | 7.02 | 130 |
| | M225L | 0.63 | 134 | 4.76 | 88 |
| | I234N | 0.52 | 111 | 5.90 | 109 |
| | D320E | 0.72 | 153 | 6.78 | 125 |
| | A347V | 2.26 | 481 | 7.75 | 143 |
| | K359Q | 0.39 | 83 | 7.40 | 137 |
| | G360R | 0.82 | 174 | 3.60 | 67 |
| | D361 G | 4.35 | 926 | 2.98 | 55 |
| | D451V | 0.53 | 113 | 5.46 | 101 |
| | Y625C | 0.43 | 91 | 6.11 | 113 |
| | H656Y | 0.49 | 104 | 6.26 | 116 |
| | S662L | 0.48 | 102 | 6.02 | 111 |

### Embodiment 2-7

### Evaluation of various mutations at position 75

The substitution of Leu for Trp at position 75 was found to greatly enhance specific activity at pH 7.5 and pH 10.0. To determine the effect of substitution of other amino acids for the Trp at position 75, site-specific mutation was conducted on the parent CGTase structural gene using primers 3 to 15 (SEQ ID NOS: 5 to 17), suitable opposing primers present within the CGTase gene, and *Pyrobest* DNA polymerase (Takara), and plasmids having genes encoding various mutant CGTases were prepared. *E. coli* JM109 that had been transformed with the plasmids prepared was cultured and the specific activity of the purified enzymes prepared was evaluated. This revealed enhanced specific activity at either one of, or both, pH 7.5 and pH 10.0 for W75A, W75C, W75E, W75F, W75H, W75I, W75K, W75M, W75Q, W75S, W75T, W75V, and W75Y. See Table 3.

[Table 3]

**Table 3 Specific activity of various CGTase at position 75**

| | | pH 7.5 | | pH 10.0 | |
|---|---|---|---|---|---|
| | | Specific activity (U/mg) | Relative value (%) | Specific activity (U/mg) | Relative value (%) |
| Comp. Ex. | Parent CGTase | 0.47 | 100 | 5.41 | 100 |
| Invention | W75A | 2.27 | 483 | 5.33 | 99 |
| | W75C | 1.70 | 362 | 4.48 | 83 |
| | W75E | 1.75 | 372 | 2.10 | 39 |
| | W75F | 1.98 | 421 | 6.30 | 116 |
| | W75H | 2.84 | 604 | 5.59 | 103 |
| | W75I | 2.87 | 611 | 6.05 | 112 |
| | W75K | 3.84 | 817 | 5.23 | 97 |
| | W75M | 4.53 | 964 | 9.73 | 180 |
| | W75Q | 3.52 | 749 | 10.85 | 201 |
| | W75S | 0.85 | 181 | 1.96 | 36 |
| | W75T | 2.64 | 562 | 5.15 | 95 |
| | W75V | 3.32 | 706 | 7.32 | 135 |
| | W75Y | 2.32 | 494 | 9.33 | 172 |

Purified CGTase and the various purified mutant CGTases were added in a ratio of 0.15 mg/g dry starch to a substrate obtained by dissolving 10 percent (w/v) soluble starch in 25 mM HEPES-NaOH (pH 7.5) with heating, and the mixture was reacted for 8 hours at 50°C. The reaction solution was maintained for 10 minutes at 100°C to deactivate the enzyme and CD-containing compositions were prepared. The sugar composition thereof was determined by HPLC. As shown in Table 4, an increase in the quantity of CD produced relative to the quantity of CD produced when the parent CGTase was employed was observed when the mutant CGTases were employed.

[Table 4]

**Table 4**

| CD composition of CD-containing composition when parent CGTase and mutant CGTases were employed | | | | |
|---|---|---|---|---|
| | | *alpha*-CD | *bet*a-CD | *gamma-*CD |
| Comp. Ex. | Parent CGTase | n. d. | n.d. | 1.2% |
| Invention | W75A | n. d. | 0.4% | 4.6% |
| | W75C | n. d. | 0.3% | 4.4% |
| | W75E | n. d. | n.d. | 3.7% |
| | W75F | n. d. | 1.3% | 4.5% |
| | W75H | n. d. | 0.9% | 4.7% |
| | W75I | n. d. | 0.6% | 5.5% |
| | W75K | n. d. | 1.1% | 4.8% |
| | W75L | n. d. | 2.3% | 4.7% |
| | W75M | n. d. | 2.0% | 4.7% |
| | W75Q | n. d. | 3.6% | 3.8% |
| | W75S | n. d. | n.d. | 2.8% |
| | W75T | n. d. | 0.3% | 5.0% |
| | W75V | n. d. | 0.6% | 5.2% |
| | W75Y | n. d. | 3.3% | 3.5% |

| | | | | |
|---|---|---|---|---|
| n.d.: Below detection threshold. | | | | |

### Embodiment 2-8

### Evaluation of various mutations at position 134

The substitution of Ala for Val at position 134 was found to greatly enhance specific activity at pH 7.5. To determine the effect of substitution of other amino acids for the Val at position 134, site-specific mutation was conducted on the parent CGTase structural gene using primers 16 to 18 (SEQ ID NOS 18 to 20), suitable opposing primers present within the CGTase gene, and *Pyrobest* DNA polymerase (Takara), and plasmids having genes encoding various mutant CGTases were prepared. *E. coli* JM109 that had been transformed with the plasmids prepared was cultured and the specific activity of the purified enzymes prepared was evaluated. This revealed enhanced specific activity at either one of, or both, pH 7.5 and pH 10.0 for V134P, V134S, and V134T. See Table 5.

[Table 5]

**Table 5 Specific activity of various CGTase mutations at position 134**

| | | pH 7.5 | | pH 10.0 | |
|---|---|---|---|---|---|
| | | Specific activity (U/mg) | Relative value (%) | Specific activity (U/mg) | Relative value (%) |
| Comp. Ex. | Parent CGTase | 0.47 | 100 | 5.41 | 100 |
| Invention | V134P | 1.99 | 423 | 8.50 | 157 |
| | V134S | 1.37 | 291 | 5.50 | 102 |
| | V134T | 0.81 | 172 | 5.26 | 97 |

Purified CGTase and the various purified mutant CGTases were added in a ratio of 0.15 mg/g dry starch to a substrate obtained by dissolving 10 percent (w/v) soluble starch in 25 mM HEPES-NaOH (pH 7.5) with heating, and the mixture was reacted for 8 hours at 50°C. The reaction solution was maintained for 10 minutes at 100°C to deactivate the enzyme and CD-containing compositions were prepared. The sugar composition thereof was determined by HPLC. As shown in Table 6, an increase in the quantity of CD produced relative to the quantity of CD produced when the parent CGTase was employed was observed when the mutant CGTases were employed.

[Table 6]

**Table 6**

| CD composition of CD-containing composition when parent CGTase and mutant CGTases were employed | | | | |
|---|---|---|---|---|
| | | *alpha*-CD | *beta*-CD | *gamma*-CD |
| Comp. Ex. | Parent CGTases | n. d. | n.d. | 1.2% |
| Invention | V134A | n. d. | 0.5% | 3.0% |
| | V134P | n. d. | 0.9% | 3.7% |
| | V134S | n. d. | n.d. | 3.5% |
| | V134T | n. d. | n.d. | 2.3% |

| | | | | |
|---|---|---|---|---|
| n.d.: Below detection threshold | | | | |

### Embodiment 2-9

### Evaluation of various mutations at position 223

To determine the effect of the substitution of amino acids other than Val at position 223, site-specific mutation was conducted on the parent CGTase structural gene using primers 19 to 21 (SEQ ID NOS 21 to 23), suitable opposing primers present within the CGTase gene, and *Pyrobest* DNA polymerase (Takara), and plasmids having genes encoding various mutant CGTases were prepared. *E. coli* JM109 that had been transformed with the plasmids prepared was cultured and the specific activity of the purified enzymes prepared was evaluated. This revealed enhanced specific activity for A223H, A223K, and A223R. See Table 7.

[Table 7]

**Table 7 Specific activity of various CGTase mutations at position 223**

| | | pH 7.5 | | pH 10.0 | |
|---|---|---|---|---|---|
| | | Specific activity (U/mg) | Relative value (%) | Specific activity (U/mg) | Relative value (%) |
| Comp. Ex. | Parent CGTase | 0.47 | 100 | 5.41 | 100 |
| Invention | A223H | 0.99 | 211 | 3.71 | 69 |
| | A223K | 1.45 | 309 | 4.45 | 82 |
| | A223R | 1.86 | 396 | 8.38 | 155 |

Purified CGTase and the various purified mutant CGTases were added in a ratio of 0.15 mg/g dry starch to a substrate obtained by dissolving 10 percent (w/v) soluble starch in 25 mM HEPES-NaOH (pH 7.5) with heating, and the mixture was reacted for 8 hours at 50°C. The reaction solution was maintained for 10 minutes at 100°C to deactivate the enzyme and CD-containing compositions were prepared. The sugar composition thereof was determined by HPLC. As shown in Table 8, an increase in the quantity of CD produced relative to the quantity of CD produced when the parent CGTase was employed was observed when the mutant CGTases were employed.

[Table 8]

**Table 8**

| CD composition of CD-containing composition when parent CGTase and mutant CGTases were employed | | | | |
|---|---|---|---|---|
| | | *alpha*-CD | *beta*-CD | *gamma-*CD |
| Comp. Ex. | Parent CGTase | n. d. | n. d. | 1.2 |
| Invention | A223H | n. d. | 0.5% | 3.2% |
| | A223K | n. d. | 0.8% | 5.1% |
| | A223R | n. d. | 0.8% | 5.4% |
| | A223V | n. d. | n. d. | 1.5% |

| | | | | |
|---|---|---|---|---|
| n.d.: Below detection threshold | | | | |

### Embodiment 2-10

### Evaluation of various mutations at position 347

The substitution of Val for Ala at position 347 was found to greatly enhance specific activity at pH 7.5 and pH 10.0. To determine the effect of substitution of other amino acids for the Ala at position 347, site-specific mutation was conducted on the parent CGTase structural gene using primers 22 to 31 (SEQ ID NOS 24 to 33), suitable opposing primers present within the CGTase gene, and *Pyrobest* DNA polymerase (Takara), and plasmids having genes encoding various mutant CGTases were prepared. *E. coli* JM109 that had been transformed with the plasmids prepared was cultured and the specific activity of the purified enzymes prepared was evaluated. This revealed enhanced specific activity at either one of, or both, pH 7.5 and pH 10.0 for A347C, A347D, A347E, A347H, A347I, A347L, A347N, A347S, A347T, and A347Y. See Table 9.

[Table 9]

**Table 9 Specific activity of various CGTase mutations at position 347**

| | | pH 7.5 | | pH 10.0 | |
|---|---|---|---|---|---|
| | | Specific activity (U/mg) | Relative value (%) | Specific activity (U/mg) | Relative value (%) |
| Comp. Ex. | Parent CGTase | 0.47 | 100 | 5.41 | 100 |
| Invention | A347C | 3.01 | 640 | 8.55 | 158 |
| | A347D | 2.23 | 474 | 5.56 | 103 |
| | A347E | 1.00 | 213 | 8.61 | 159 |
| | A347H | 0.84 | 179 | 4.25 | 79 |
| | A347I | 3.31 | 704 | 9.98 | 184 |
| | A347L | 2.78 1 | 59 | 6.60 | 122 |
| | A347N | 1.43 | 304 | 5.34 | 99 |
| | A347S | 0.61 | 130 | 5.43 | 100 |
| | A347T | 1.49 | 317 | 7.19 | 133 |
| | A347Y | 5.47 | 1164 | 9.88 | 183 |

Purified CGTase and the various purified mutant CGTases were added in a ratio of 0.15 mg/g dry starch to a substrate obtained by dissolving 10 percent (w/v) soluble starch in 25 mM HEPES-NaOH (pH 7.5) with heating, and the mixture was reacted for 8 hours at 50°C. The reaction solution was maintained for 10 minutes at 100°C to deactivate the enzyme and CD-containing compositions were prepared. The sugar composition thereof was determined by HPLC. As shown in Table 10, an increase in the quantity of CD produced relative to the quantity of CD produced when the parent CGTase was employed was observed when the mutant CGTases were employed.

[Table 10]

**Table 10 CD composition of CD-containing compositions employing parent CGTase and mutant CGTase**

| | | *alpha*-CD | *beta*-CD | *gamma*-CD |
|---|---|---|---|---|
| Comp. Ex. | Parent CGTase | n. d. | n. d. | 1.2% |
| Invention | A347C | n. d. | 2.6% | 5.4% |
| | A347D | n. d. | n. d. | 3.7% |
| | A347E | n. d. | n. d. | 3.7% |
| | A347H | n. d. | 2.3% | 5.3% |
| | A347I | n. d. | 0.8% | 4.6% |
| | A347L | n. d. | 0.7% | 5.1% |
| | A347N | n. d. | n. d. | 3.3% |
| | A347S | n. d. | 0.5% | 4.9% |
| | A347T | n. d. | 2.7% | 5.6% |
| | A347V | n. d. | 1.2% | 4.4% |
| | A347Y | n. d. | 1.9% | 5.5% |

| | | | | |
|---|---|---|---|---|
| n.d.: Below detection threshold | | | | |

### Embodiment 2-11

### Evaluation of various mutations at position 361

The substitution of Gly for Asp at position 361 was found to greatly enhance specific activity at pH 7.5. To determine the effect of substitution of other amino acids for the Asp at position 361, site-specific mutation was conducted on the parent CGTase structural gene using primers 32 to 48 (SEQ ID NOS 34 to 50), suitable opposing primers present within the CGTase gene, and *Pyrobest* DNA polymerase (Takara), and plasmids having genes encoding various mutant CGTases were prepared. *E. coli* JM109 that had been transformed with the plasmids prepared was cultured and the specific activity of the purified enzymes prepared was evaluated. This revealed enhanced specific activity at either one of, or both, pH 7.5 and pH 10.0 for D361A, D361C, D361E, D361 F, D361 H, D361I, D361K, D361 L, D361 M, D361 N, D361 P, D361 Q, D361 S, D361T, D361V, D361W, and D361Y. See Table 11.

[Table 11]

**Table 11 Specific activity of various CGTase mutations at position 361**

| | | pH 7.5 | | pH 10.0 | |
|---|---|---|---|---|---|
| | | Specific activity (U/mg) | Relative value (%) | Specific activity (U/mg) | Relative value (%) |
| Comp. Ex. | Parent CGTase | 0.47 | 100 | 5.41 | 100 |
| Invention | D361A | 5.14 | 1094 | 7.51 | 139 |
| | D361C | 0.74 | 157 | 1.73 | 32 |
| | D361E | 0.60 | 128 | 9.04 | 167 |
| | D361F | 2.27 | 483 | 4.42 | 82 |
| | D361H | 2.52 | 536 | 4.02 | 74 |
| | D361I | 3.30 | 702 | 7.22 | 133 |
| | D361K | 1.63 | 347 | 1.51 | 28 |
| | D361L | 3.36 | 715 | 5.28 | 98 |
| | D361M | 1.68 | 357 | 3.53 | 65 |
| | D361N | 3.38 | 719 | 4.23 | 78 |
| | D361P | 6.30 | 1340 | 7.28 | 135 |
| | D361Q | 2.76 | 587 | 4.12 | 76 |
| | D361S | 4.86 | 1034 | 8.23 | 152 |
| | D361T | 3.59 | 764 | 8.82 | 163 |
| | D361V | 6.29 | 1338 | 11.43 | 211 |
| | D361W | 1.62 | 345 | 3.72 | 69 |
| | D361Y | 1.14 | 243 | 1.32 | 24 |

Purified CGTase and the various purified mutant CGTases were added in a ratio of 0.15 mg/g dry starch to a substrate obtained by dissolving 10 percent (w/v) soluble starch in 25 mM HEPES-NaOH (pH 7.5) with heating, and the mixture was reacted for 8 hours at 50°C. The reaction solution was maintained for 10 minutes at 100°C to deactivate the enzyme and CD-containing compositions were prepared. The sugar composition thereof was determined by HPLC. As shown in Table 12, an increase in the quantity of CD produced relative to the quantity of CD produced when the parent CGTase was employed was observed when the mutant CGTases were employed.

[Table 12]

**Table 12 CD composition of CD-containing compositions obtained using parent CGTase and mutant CGTase**

| | | *alpha*-CD | *beta*-CD | *gamma-*CD |
|---|---|---|---|---|
| Comp. Ex. | Parent CGTase | n. d. | n.d. | 1.2% |
| Invention | D361A | n. d. | n.d. | 5.1% |
| | D361C | n. d. | n.d. | 2.2% |
| | D361E | n. d. | n.d. | 1.9% |
| | D361F | n. d. | 0.3% | 4.1% |
| | D361G | n. d. | 0.2% | 5.2% |
| | D361H | n. d. | 0.2% | 4.1% |
| | D361I | n. d. | n.d. | 4.4% |
| | D361K | n. d. | n.d. | 2.8% |
| | D361L | n. d. | 0.2% | 4.4% |
| | D361M | n. d. | 0.2% | 3.2% |
| | D361N | n. d. | 0.2% | 4.1% |
| | D361P | n. d. | 0.7% | 6.3% |
| | D361Q | n. d. | 0.5% | 4.2% |
| | D361S | n. d. | n.d. | 5.3% |
| | D361T | n. d. | n.d. | 4.5% |
| | D361V | n. d. | 0.2% | 5.5% |
| | D361W | n. d. | 0.3% | 2.9% |
| | D361Y | n. d. | n.d. | 2.7% |

| | | | | |
|---|---|---|---|---|
| n.d.: Below detection threshold | | | | |

### Embodiment 2-12

### Evaluation of specific activity of mutants at various pH levels

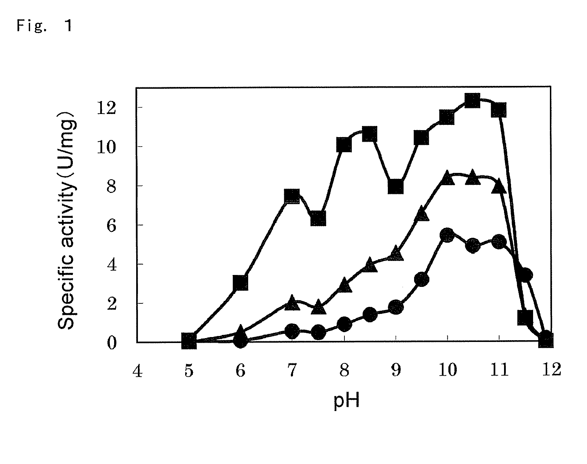

The specific activity of parent CGTase (● in Fig. 1), A223R (▲ in Fig. 1), and D361V (■ in Fig. 1) was evaluated at pH 5 to 11.9. The results are given in Fig. 1. As a result, increased specific activity at pH 6 to 11 was observed for both mutations A223R and D361V.

### [Industrial Applicability]

The present invention is useful in the domain of manufacturing gamma-CD and gamma-CD-containing compositions.

### [Brief Description of Drawings]

Fig. 1 shows the results of evaluation of the specific activity at various pH levels (pH 5 to 11.9) of the mutants obtained in Embodiment 2-12.

## Claims

1. A mutant cyclodextrin glucanotransferase, having greater specific activity in the synthesis of *gamma*-cyclodextrin than the parent *gamma*-cyclodextrin glucanotransferase, in which at least one of the amino acid residues in the parent *gamma*-cyclodextrin glucanotransferase has been replaced with an amino acid residue differing from that particular amino acid residue in the parent *gamma-*cyclodextrin glucanotransferase.

2. The mutant according to Claim 1, wherein in the parent *gamma-*cyclodextrin glucanotransferase having the amino acid sequence denoted by SEQ ID NO: 1 in the Sequence Listing, at least one of the amino acid residues at positions 75, 82, 91, 92, 119, 134, 147, 151, 223, 225, 234, 320, 347, 359, 360, 361, 451, 625, 656, and 662 in the amino acid sequence denoted by SEQ ID NO: 1 in the Sequence Listing has been replaced with an amino acid residue differing from the amino acid residue indicated for the corresponding position in the amino acid sequence of SEQ ID NO: 1.

3. The mutant according to Claim 2, wherein the differing amino acid residue(s) is/are:
Ala, Cys, Gln, Glu, His, Ile, Leu, Lys, Met, Phe, Ser, Thr, Tyr, or Val at position 75;
Asp at position 82;
Leu at position 91;
Thr at position 92;
Val at position 119;
Ala, Pro, Ser, or Thr at position 134;
Thr at position 147;
Asp at position 151;
Arg, His, Lys, or Val at position 223;
Leu at position 225;
Asn at position 234;
Glu at position 320;
Asn, Asp, Cys, Glu, His, Ile, Leu, Ser, Thr, Tyr, or Val at position 347;
Gln at position 359;
Arg at position 360;
Ala, Asn, Cys, Gln, Gly, Glu , His, Ile, Leu, Lys, Met, Phe, Pro, Ser, Thr, Trp, Tyr, or Val at position 361;
Val at position 451;
Cys at position 625;
Tyr at position 656; and
Leu at position 662.

4. The mutant according to Claim 2 or 3, wherein in the amino acid sequence denoted by SEQ ID NO: 1 in the Sequence Listing, one or a few deletions, substitutions, or additions have been made to amino acid residues other than those at positions 75, 82, 91, 92, 119, 134, 147, 151, 223, 225, 234, 320, 347, 359, 360, 361, 451, 625, 656, and 662.

5. The mutant according to Claim 1, wherein in the parent *gamma-*cyclodextrin glucanotransferase having an amino acid sequence having 60 percent or greater homology with the amino acid sequence denoted by SEQ ID NO: 1 of the Sequence Listing, at least one amino acid residue among the amino acid residues corresponding to positions 75, 82, 91, 92, 119, 134, 147, 151, 223, 225, 234, 320, 347, 359, 360, 361, 451, 625, 656, and 662 in the amino acid sequence of the parent *gamma*-cyclodextrin glucanotransferase denoted by SEQ ID NO: 1 in the Sequence Listing has been replaced with an amino acid residue differing from the amino acid residue at the corresponding position in the amino acid sequence of the parent *gamma*-cyclodextrin glucanotransferase.

6. The mutant according to any one of Claims 1 to 5, wherein the specific activity of which as measured at a pH between pH 6 to 11, is 1.1-fold or greater the specific activity of the parent *gamma*-cyclodextrin glucanotransferase.

7. The mutant according to any one of Claims 1 to 6, wherein the specific activity of which as measured at pH 7.5 or pH 10, is 1.1-fold or greater the specific activity of the parent *gamma*-cyclodextrin glucanotransferase.

8. A gene encoding the amino acid sequence of the mutant according to any one of Claims 1 to 7.

9. A recombinant vector comprising the gene according to Claim 8.

10. A transformant that has been transformed or modified by chromosomal homologous recombination with the recombinant vector according to Claim 9.

11. A method for manufacturing the mutant according to any one of Claims 1 to 7 comprising: culturing the transformant according to Claim 10, and collecting the mutant cyclodextrin glucanotransferase produced by the transformant.
